(19) Europäisches Patentamt
European Patent Office
Office européen des brevets

(11) EP 3 093 280 A1

(12) **EUROPEAN PATENT APPLICATION**

(43) Date of publication:
**16.11.2016 Bulletin 2016/46**

(21) Application number: **15167707.7**

(22) Date of filing: **13.05.2015**

(51) Int Cl.:
*C07C 2/32* (2006.01)
*C07C 11/02* (2006.01)
*B01J 31/12* (2006.01)
*B01J 31/14* (2006.01)
*B01J 31/18* (2006.01)
*C07F 7/00* (2006.01)
*C07C 2/88* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Sasol Performance Chemicals GmbH**
**20537 Hamburg (DE)**

(72) Inventors:
- **Boddien, Albert**
  **25524 Itzehoe (DE)**
- **Kempe, Rhett**
  **95444 Bayreuth (DE)**
- **Kretschmer, Winfried**
  **95466 Weidenberg (DE)**
- **Obenauf, Johannes**
  **37154 Northeim (DE)**

(74) Representative: **Müller Schupfner & Partner**
**Patent- und Rechtsanwaltspartnerschaft mbB**
**Schellerdamm 19**
**21079 Hamburg (DE)**

Remarks:
The application is published incomplete as filed (Rule 68(1) EPC).

# (54) PROCESS FOR THE OLIGOMERISATION OF OLEFINS BY COORDINATIVE CHAIN TRANSFER POLYMERISATION AND CATALYST SYNTHESIS

(57) The present invention relates to a process for the oligomerisation of olefins, in particular ethylene, via coordinative chain transfer polymerisation (CCTP) and alkyl elimation reaction. The present invention further relates to a new method of synthesizing CCTP-catalysts. A preferred embodiment of the invention relates to CCTP of olefins, in particular ethylene, with the use of guanidinato or amidinato complexes of titanium zirconium or lanthanides, a chain displacement catalyst (CDC) such as a nickel or cobalt compound and a chain transfer agent (CSA) such as a main group metal alkyl.

**Fig. 1**

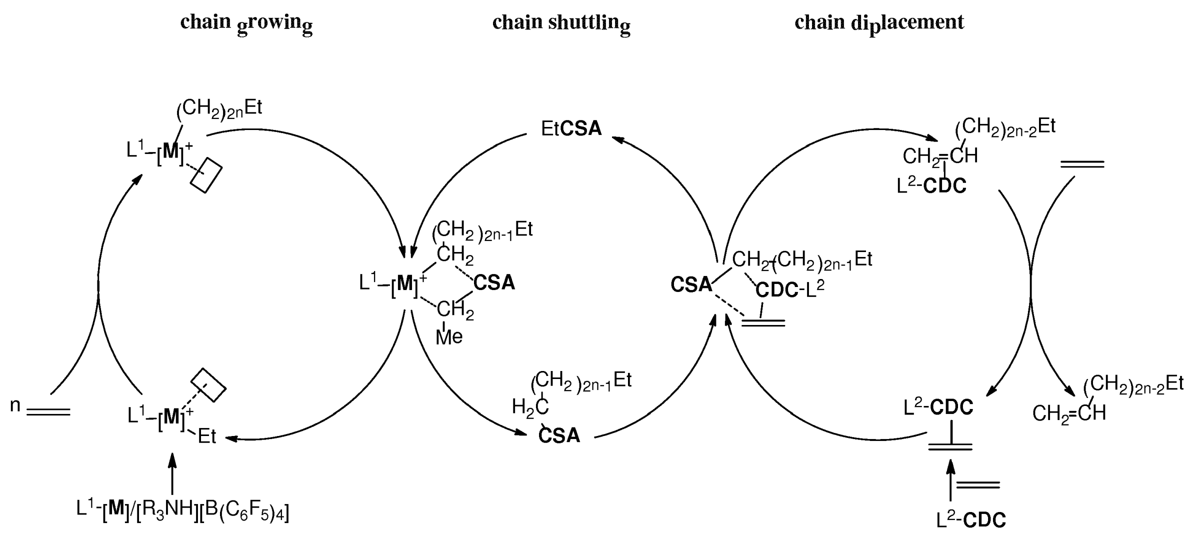


EP 3 093 280 A1

## Description

INTRODUCTION

The present invention relates to a process for the oligomerisation of olefins, in particular ethylene, via coordinative chain transfer polymerisation (CCTP) and alkyl elimation reaction. The present invention further relates to a new method of synthesizing CCTP-catalysts. A preferred embodiment of the invention relates to CCTP of olefins, in particular ethylene, with the use of guanidinato or amidinato complexes of titanium zirconium or lanthanides, a chain displacement catalyst (CDC), such as a nickel or cobalt compound,; and a chain transfer agent (CSA), typically a main group metal alkyl. The characteristics of the process according to the invention are that various olefins can be produced either at a high stoichiometric excess of CSA but also using only catalytic amounts of CSA. Further by changing the parameters of the process Schulz-Flory or Poisson distributed olefins can be obtained as wanted.

BACKGROUND OF THE INVENTION AND PRIOR ART DISCUSSION

The oligomerisation of olefins can yield product distributions with regard to chain lengths which are either Poisson distributions or Schulz-Flory distributions. A Poisson distribution is characterized by the formula $X_p = (x^p \cdot e^{-x}) / p!$, and a Schulz-Flory distribution by the formula $X_p = \beta(1+\beta)^{-p}$, whereas $X_p$ is the mole fraction with p added olefins, x is the Poisson distribution coefficient equal to the average number of olefin molecules added per M-C bond, and ß is the Schulz-Flory distribution coefficient. A Poisson distribution is a normal distribution curve approximately centered about the average degree of oligomerisation. Schulz-Flory distribution describes a product distribution having a greater molar amount of the small oligomers with a broader range of chain lengths. For short chain oligomers C<12 mainly Schulz-Flory distributions are desired, however, if chain length above C12 are requested Poisson distributed products are often desired.

Linear alpha olefins (LAOs) are valuable commodity chemicals used as precursors in many areas of industry. Annually, more than 3 Mio. tons of alpha olefins are produced globally. In addition, linear alpha-olefins are used as such in many final products in various applications. For example, the light olefin fractions, 1-butene, 1-hexene and 1-octene, are used as co-monomers in the rapidly growing polymer market, in particular for the production of LLDPE (Linear Low Density Polyethylene) and EPDM (Ethylene Propylene Diene Monomer) rubber. The middle olefin fractions, such as 1-decene, 1-dodecene and 1-tetradecene are raw materials for the production of synthetic oils, detergents and shampoos. The heavy olefin fractions can be used as additives for lubricating oils, surfactants, oil field chemicals, waxes and as polymer compatibilisers.

Commonly, commercial LAO plants produce even-numbered alpha-olefins via ethylene oligomerisation. Different thereto Sasol Chemical Industries produces 1-hexene, 1-octene and also smaller quantities of 1-pentene via Fischer-Tropsch-Synthesis from coal. Here, either the Coal-to-Liquid-process (CtL-process) or the Gas-to-Liquid-process (GtL-process) can be used. In the CtL-process, the coal is first reacted at very high temperatures (above 1000°C) with water vapour and oxygen to form synthesis gas which, subsequent to the separation of nitrogen oxides and sulphur dioxide, is reacted via heterogeneous catalysis to form hydrocarbons including alpha-olefins and water. In the GtL-process, natural gas is reacted via addition of oxygen and water vapour to form synthesis gas, and the latter is transformed into hydrocarbons in a Fischer-Tropsch-Synthesis. Both processes have the disadvantage that also a broad variety of byproducts (paraffins and alcohols) are produced. This means that more pure alpha-olefins become accessible only after purification processes (e.g. DE 10022466 A1). Other industrial-scale procedures for the preparation of $\alpha$-olefins are the cracking of paraffins, the dehydrogenation of paraffins and the dehydration of alcohols, decarboxylation of lactones and fatty acids, or chain growth reactions including the oligomerisation of ethylene (e.g. US 20140155666A1). Since ethylene represents an easily available raw material, the first mentioned methods of production play a minor role. The vast majority of alpha-olefins are produced via oligomerisation of ethylene providing exclusively olefins with an even number of C-atoms which have the highest value for commercial applications (e.g. G. J. P. Britovsek et al., Angew. Chem. Int. Ed. 1999, 38, 428-447; S. D. Ittel et al., Chem. Rev. 2000, 100, 1169-1204). Well known industrially used production processes for LAO's, which are based on the oligomerisation, of ethylene, are the following:

- the oligomerisation reaction in the Shell Higher-Olefin-Process (SHOP;) using a nickel complex, providing exclusively a Schulz-Flory distribution from the oligomerisation reaction;

- the alpha-Sablin-process which uses a zirconia (Zr) compound in conjunction with an aluminium alkyl co-catalyst, providing exclusively a Schulz-Flory distribution from the oligomerisation reaction;

- the IDEMITSU's ethylene oligomerisation process (LINEALENE), which make use of a zirconium based catalyst system in combination with triethyl aluminium as co-catalyst, providing exclusively a Schulz-Flory distribution from

the oligomerisation reaction;

- the Chevron-Phillips-Gulf-process (Gulftene - Schulz-Flory distribution) and the INEOS-ethyl-process (POISSON distribution) which rely on the aluminium alkyl mediated oligomerisation of ethylene and the subsequent nickel (Ni)-catalyzed displacement of the olefins.

The above mentioned processes yield a broad distribution of molecules having different chain lengths. It is in particular difficult to produce certain chain lengths of LAO's, having a carbon chain number beyond 20 carbon atoms (C20) in an economic feasible manner. Due to the constraints of the standard oligomerisation reaction the products have more branching in the higher range alpha olefins. Hence, the distribution is rather inflexible and only a few percent of C20+ material (higher oligomers) can be obtained. All known processes for producing alpha olefin at an industrial scale result either in a Schulz-Flory or in a Poisson distribution and cannot be controlled to change from one type of distribution to the other.

In addition to the above processes, there are processes which selectively produce a single alpha-olefin in high purity. They include the Chevron-Phillips trimerisation process for the production of 1-hexene, the Sasol tri- and tetramerisation process yielding 1-hexene and 1-octene and the Axens/Sabic Alphabutol-process yielding 1-butene.

However, a need remains for processes, which are easy tuneable and provide variable distributions of alpha-olefins at mild reaction conditions and with a high yield.

A great variety of catalysts capable of catalysing coordinative chain transfer polymerisation (CCTP) have been proposed in the literature. CCTP is commonly used to control and modify molecular weights of high molecular weight polymers with molecular weights of above 10000 g/mol of the products so obtained. These transition metal based catalysts are typically used together with co-catalysts which usually act as chain transfer/shuttling agent (CSA). Suitable co-catalysts include alkyl zinc, alkyl aluminium, alkyl aluminium halides and alkyl alumoxanes, commonly used together with inert, non-coordinating ion forming compounds (activators), Lewis and Bronstedt acids and mixtures thereof. Such prior art processes are disclosed in US 5276220; G. J. P. Britovsek et al., Angew. Chem. Int. Ed. 2002, 41, 489-491; WO 2003/014046 A1; W. P. Kretschmer et al., Chem. Eur. J. 2006, 12, 8969-8978; S. B. Amin, T. J. Marks, Angew. Chem. 2008, 120, 2034-2054; I. Haas et al., Organometallics 2011, 30, 4854-4861; and A. Valente et al., Chem. Rev. 2013, 113, 3836-3857; S. K. T. Pillai et al., Chem. Eur. J. 2012, 18, 13974 - 13978; J. Obenauf et al., Eur. J. Inorg. Chem. 2013, 537-544, EP 2671639 A1 (for zirconium), W. P. Kretschmer et al., Dalton Trans., 2010, 39, 6847-6852 (for lanthanides).

One characteristics of CCTP is that polymer chains are end-capped with the respective main group metal of the co-catalyst and can be further functionalized (M. Bialek, J. Polym. Sci.: Part A: Polym. Chem. 2010, 48, 3209-3214 and W. P. Kretschmer et al., Dalton Trans. 2010, 39, 6847-6852). Nearly all catalytic systems reported until today suffer from ligand transfer from the CCTP catalyst complex onto the CSA and are therefore not stable at high CSA concentrations. However, in order to apply such catalysts systems economically, it is of outmost importance to make high CSA to CCTP ratios possible, since the CSA have to be transformed into the final product (paraffin, olefin, alcohol).

CCTP typically requires the use of a metal complex as catalyst, a co-catalyst and optionally an activator. In the understanding of the present invention the co-catalyst is a chain shuttling agent (CSA) and may optionally but not necessarily be an activator at the same time.

The activator may be for example a compound different from the chain transfer agent that is not functioning as a chain shuttling agent. Such activator is in the understanding of the invention solely called an activator and not a co-catalyst.

In order to obtain an olefin from such processes a chain displacement catalyst (CDC) has to be applied. A typical chain displacement catalyst capable of catalysing an olefin exchange reaction (beta-H elimination) is for example $Ni(acac)_2$, which is reported to give linear alpha-olefins such as disclosed in US 4918254, US 6444867, US 5780697 and US 5550303.

All processes known from the prior art apply a stoichiometric amount of main group metal alkyls. Hence, the disclosed processes are in a need to operate via a two step process carried out in two different reactors.

In EP2671639 A1 a novel guanidinato group 4 metal catalyst system is described, which catalyses the chain growth on aluminium via CCTP.

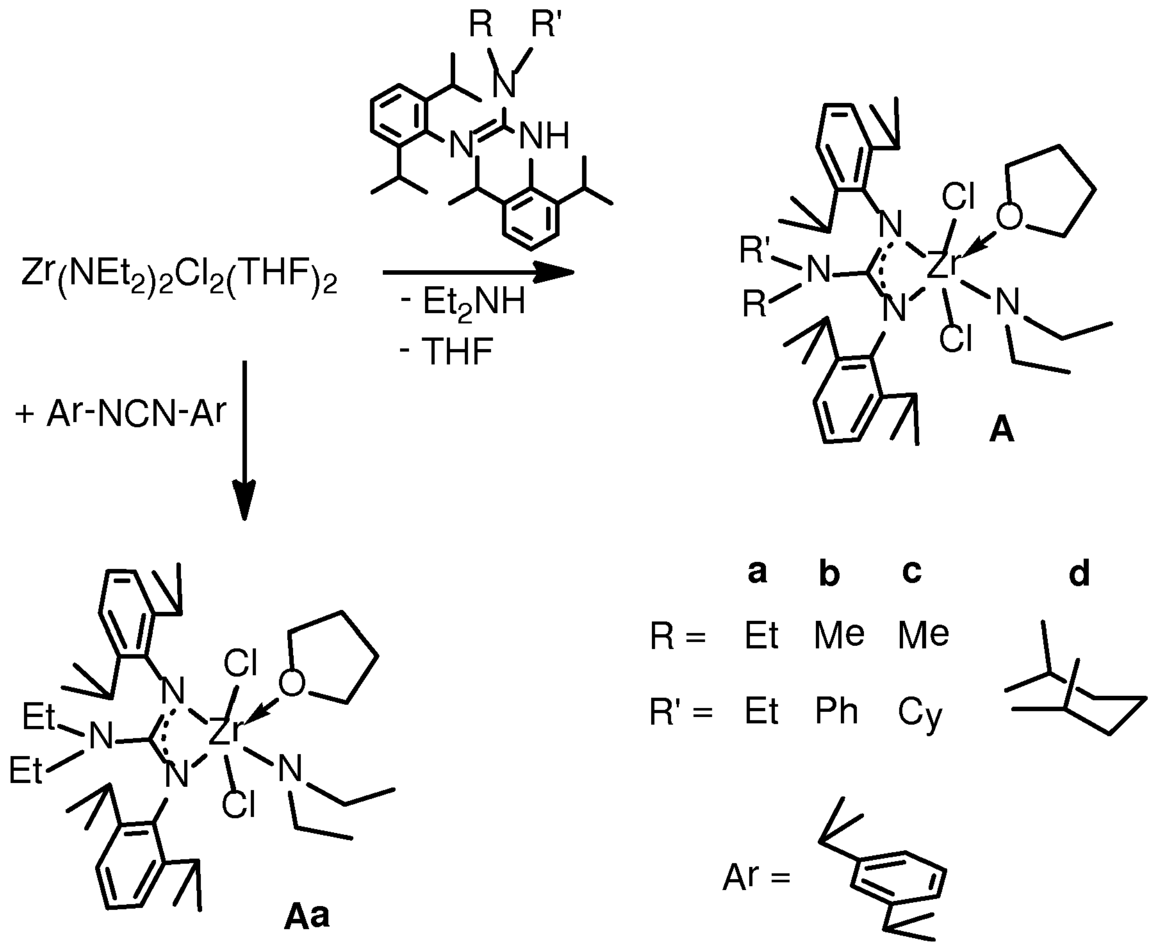


The {*N',N''*-bis[2,6-di(isopropyl)phenyl]-*N,N*-diethyl-guanidinato}-(diethylamido)-dichlorido-zirconium(VI); **Aa** {$[Et_2NC(2,6\text{-}Pr^i{}_2C_6H_3N)_2](Et_2N)ZrCl_2(THF)$; catalyst was prepared from (Z)-2,3-bis(2,6-diisopropylphenyl)-1,1-diethylguanidine or Bis(2,6-diisopropylphenyl)carbodiimide by reaction with Bis(N,N-diethylamido)-dichlorido-zirconium(IV)-bis(tetrahydrofuran), $(Et_2N)_2ZrCl_2(THF)_2$, in situ. Unfortunately, applying this in-situ complex side product formation is increased.

OBJECT OF THE PRESENT INVENTION

The object of the present invention is to find a highly flexible process which is capable of oligomerising or co-oligomerising alpha-olefins, preferably as wanted in either Poisson- or a Schulz-Flory-distribution, at very mild process conditions and with very high catalyst activities over a wide range of CSA amounts. In addition, a process is needed for the in-situ generation of alpha-olefins with the use of known CCTP catalysts systems, which so far have not been stable at high ratios of CCTP to CSA. It is a further object of the present invention to provide via an easy synthesis well-defined catalysts in a high yield.

SUMMARY OF THE INVENTION

The present invention is defined by the independent claims. Preferred embodiments are disclosed in the subordinate claims or described hereunder.

The present invention is concerned with a process capable for producing differently distributed oligomerised olefins, including linear olefins, branched olefins, alpha- olefins and/or internal olefins), particular linear olefins at mild condition, in a flexible manner. In accordance with this invention a process is provided for preparing linear and/or branched oligomerised olefins, particularly linear alpha-olefins including waxes.

The invention makes use of a CCTP catalyst system operating preferably at temperatures of 20-200 °C which comprises a metal organic complex capable of oligomerising or co-oligomerising alpha-olefins as gases or liquids, an activator, a chain shuttling agent, which is capable of transferring the alkyl chain at the catalyst onto the chain shuttling agent, and a chain-displacement-catalyst (CDC) capable of catalysing the beta-H-elimination and if necessary isomerisation to finally obtain olefins (alpha and/or internal olefins) with a controlled chain length distribution. The oligomerisation according to the present invention can be conducted at high CCTP to CSA and low CCTP to CSA ratios. Surprisingly the problem of lack of stability of many CCTP catalyst systems (in particular at ratios of CCTP / CSA 1 to above 50000) can be superseded in the presence of a CDC, in particular at a ratio of CCTP / CDC of 1: 1 and above, as defined herein.

An advantage of the in situ use of the two catalysts is that CDC can be used either in stoichiometric or catalytic amounts. The obtained olefins can vary in chain length and distribution, which depends on CCTP, CSA, CDC, ratios and the process conditions applied. Preferably, the oligomerised olefins obtained are C4 to C80 olefins, most preferably C16 to C30 olefins.

Further, a new synthesis route was found for a preferred class of CCTP catalysts. It was surprisingly found that switching from $(Et_2N)_2ZrCl_2(THF)_2$ to (N,N-diethylamido)-trichlorido-zirconium(IV) and $(Et_2N)ZrCl_3(OEt_2)$ as the starting material provides a method for preparing Zirconium trialkyl complexes of for example (*Z*)-2,3-bis(2,6-diisopropylphenyl)-1,1-dialkylguanidines in crystalline form and high yields. As an intermediate {*N',N"*-bis[2,6-di(isopropyl)phenyl]-*N*,*N*-dialkyl-guanidinato}-(diethylamino)-trichlorido-zirconium(VI) complexes are obtained which upon reaction with a Grignard reagent provide the desired compounds. The new synthesis route enables the synthesis of group 4 metal catalyst alkyl complexes, which are about 50 % more active as their chloride congeners.

Subject of the present invention is a highly flexible tandem catalytic approach to produce alpha-olefins with a Schulz-Flory and/or Poisson distribution. As presented in scheme 1 (Fig.1), a CCTP catalyst M is used to catalyze the oligomerisation/polymerisation of ethylene or ethylene and propylene and/or C4- to C8- olefins and subsequently transfers the carbon chain onto the main group metal alkyl CSA (chain shuttling agent). This CSA than shuttles the carbon chain to a chain displacement catalyst (CDC), which exchanges the carbon chain for an alkyl group by releasing one equivalent of an alpha-olefin. Fig. 1 illustrates the assumed mechanism of the tandem catalyst oligomerisation process of ethylene.

The invention is systematically described by the following listing of items:

Item 1: Process for the manufacture of oligomerised olefins by bringing in contact with each other

I Simultaneous Process:

(a) one or more C2 to C8 olefins,
(b) a coordinative chain transfer polymerisation catalyst (CCTP catalyst) comprising one or more organo metallic transition metal compounds and one or more ligands,
(c) a chain shuttling agent (CSA) being one or more metal alkyls selected from the group II, XII and XIII,
(d) a chain displacement catalyst (CDC) being one or more member selected from the group consisting of a nickel salt, a cobalt salt, an organo metallic nickel complex and an organo metallic cobalt complex,
to form a growth composition thereby obtaining oligomerised olefins having an oligomerisation degree of 2 to 100,

II Sequential Process:
wherein the process additionally includes that (d) is brought in contact only at a later point in time with the growth composition when the oligomerisation has commenced or has come to an end and (b) is at least partially or completely transformed into an inactive reaction product or inactive degradation product.
Preferred is to carry out only the simultaneous process.

Item 2: The process according to item 1, wherein the growth composition further comprises an activator for the coordinative chain transfer polymerisation catalyst (CCTP catalyst) being an aluminium or boron containing compound comprising at least one hydrocarbyl group.

Item 3: The process according to item 1 or item 2 , wherein the olefin is one or more member selected from the group consisting of ethylene, propylene, 1-butene, 1-pentene and 1-hexene, preferably one or more member selected from the group ethylene, propylene or ethylene and propylene.

Item 4: The process according to one or more of the preceding items, wherein the one or more organo metallic transition metal compounds comprises one or two transition metals, preferably one transition metal, selected from group III, group IV, preferably Ti or Zr, most preferably Zr, group V, group VI, group IX or group X, of the periodic table (according to IUPAC).

Item 5: The process according to one or more of the preceding items, wherein one or two ligands are selected from cyclopentadienyl, indenyl, fluorine, diamide ligands, phenoxy-imine-ligand, indolide-imine-ligands, amidinate, guanidinate, amidopyridine, pyrrdinimine and alcoholate each optionally substituted.

Item 6: The process according to one or more of the preceding items, wherein the CCTP catalyst is deactivated during or after the oligomerisation by heating the growth composition, most preferably above 120°C or by bringing the CCTP catalyst in contact with a catalyst poison, preferably being a halogen containing compound, preferably an halogened hydrocarbyl aluminium.

Item 7: The process according to any one of the preceding items wherein the chain shuttling agent (CSA) is a C1 to C30 hydrocarbyl metal compound, methylalumoxane or both, the metal being aluminium, zinc, magnesium, indium or gallium, preferably trihydrocarbyl aluminium, dihydrocarbyl magnesium or dihydrocarbyl zinc.

Item 8: The process according to any one of the preceding items wherein the chain displacement catalyst (CDC) is selected from nickel halogenides, cobalt halogenides, nickel cyclooctadiene, cobalt cyclooctadiene, nickel acetylactonate, C1 to C30 carboxylic acid salts of nickel and mixtures thereof.

Item 9: The process according to any one of items 2 to 8 wherein the activator is methyl aluminoxan, or a perfluorated aluminate or a boron containing compound or combinations thereof and the boron containing compound preferably comprises one or more members selected from the group consisting of tris(pentafluoro phenyl) borane, tetrakis(pentafluoro phenyl) borate, tris(tetrafluoro phenyl) borane and tetrakis(tetrafluoro phenyl) borate.

Item 10: The process according to any one of the preceding items wherein the molar ratio of the coordinative chain transfer polymerisation catalyst (CCTP catalyst) to the chain shuttling agent (CSA) is 1 : > 50000 or 1 : 50 to 1 : 10000, except for methyl alumoxane as the CSA.

Item 11: The process according to any one of the preceding items wherein the molar ratio of the coordinative chain transfer polymerisation catalyst (CCTP catalyst) to the chain displacement catalyst (CDC)

- prior or during the oligomerisation is 1 : 0.5 to 1 : 50, preferably 1 : 1 to 1 : 2, and
- after the oligomerisation the concentration of the chain displacement catalyst (CDC) is between 1 to 10000 ppm, preferably 1 to 100 ppm (w/w) relative to the growth composition.

Item 12: The process according to any one of items 2 to 11 wherein the molar ratio of the coordinative chain transfer polymerisation catalyst (CCTP catalyst) to the activator is 1 : 1 to 1 : 4, preferably 1: 1,05 to 1: 2, except for methyl alumoxane.

Item 13: The process according to any one of the preceding items wherein the growth composition further comprises a liquid reaction medium, the liquid reaction medium comprising aromatic hydrocarbons, particularly toluene, linear and/or branched C4 to C20 hydrocarbons and mixtures thereof; cyclic and alicyclic hydrocarbons such as cyclohexane, cycloheptane, and/or methylcyclohexane,.

Item 14: The process according to any one of the preceding items wherein the reaction is carried out at a C2 to C3 pressure of 0,2 to 60 bar, preferably 1 to 20 bar; most preferably 1 to 10 bar or a C4 pressure of 0,2 to 20 bar, preferably 1 to 10 bar.

Item 15: The process according to any one of the preceding items wherein the reaction is carried out at a temperature 20 to 200°C, preferably 50 to 100°C.

Item 16: The process according to one or more of the preceding items, wherein the coordinative chain transfer polymerisation catalyst comprises as transition metal Ti, Zr or Hf and one ligand per metal of the following formula

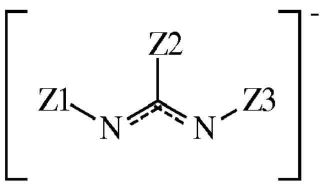

I,

the ligand being bound to the metal, wherein

Z1, Z2 and Z3 = are independently hydrocarbon or heteroatom containing hydrocarbon moieties, wherein the heteroatom, if present, for Z1 or Z3 is not directly adjacent to the N-atom and, wherein Z1, Z2 and Z3 independently from each other are optionally linked with one or more of each other.

Item 17: The process according to one or more of the preceding items, wherein the coordinative chain transfer polymerisation catalyst comprises as transition metal Ti, Zr or Hf and one ligand per metal having the following substructural formula

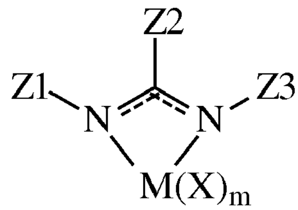

wherein

Z1 ,Z3 = each are independently from each other a di-ortho substituted aromatic moiety, each being independently hydrocarbon moieties or heteroatom containing hydrocarbon moieties, wherein the heteroatom, if present, is not directly adjacent to the N-atom,

Z2 = is a hydrocarbon moiety or a heteroatom containing hydrocarbon moiety, Z1, Z2 and Z3 independently from each other are optionally linked with one or more of each other, and

M = Titanium. Zirconium of Hafnium.

Item 18: The process of item 17 wherein Z2 is NR1 R2 with R1 and R2 independently from each other are C1 to C40 hydrocarbon moieties, optionally comprising one or more heteroatoms.

Item 19: The process according to any one of the preceding items, wherein the coordinative chain transfer polymerisation catalyst (CCTP catalyst) or its active species comprises an M - $AlR_3$ group with M = transition metal and R = C1 to C6 hydrocarbyl.

Item 20: A process for the manufacture of a Di-μ-halogen-bridged bis guanidinato tetrahalogen di zirconium compound comprising the following steps:

- bringing together
- a zirconium amido compound having the following formula

$$Zr\ (Hal)_3\ (NR^1R^2)\ Etherate$$

wherein

Hal is independent from each other Halogen, in particular Cl;

$R^1$,$R^2$ being C1 to C40 hydrocarbyl-, optionally comprising one or more heteroatoms, wherein the heteroatom is not adjacent to the N-Atom;

the etherate preferably being a di-(C1- to C6-)hydrocarbylether , in particular a di(C1- to C6-)alkylether, a di-(C2- or C3-)hydrocarbylether, in particular a Di(C2- or C3)alkylether;

and

an carbodiimid-compound having the following formula

$$(R^3)_xAr\text{-}N\text{=}C\text{=}N\text{-}Ar(R^4)_y$$

wherein

$R^3$,$R^4$= independent from each x, y is hydrocarbyl, in particular alkyl, or halogen, wherein R is preferably substituted at the 2 or 6 position of the aryl, and further wherein R is branched at the 2-position

x,y = 0 to 3 independent of each other;

Ar = is Aryl, in particular Benzene.

in a solvent.

Item 21: The process according to item 20 wherein the Di-μ-halogen-bridged bis guanidinato tetrahalogen di zirconium compound is

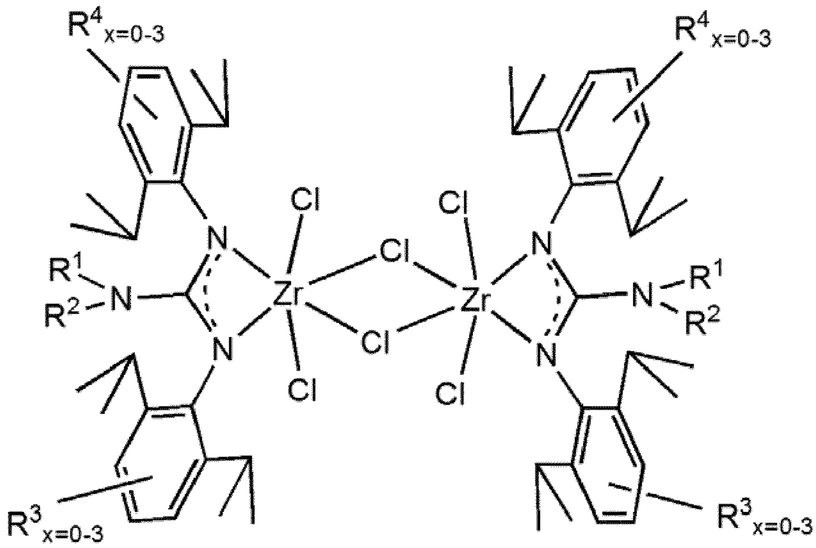

.

with

$R^1$,$R^2$ being C1 to C40 hydrocarbyl-, optionally comprising one or more heteroatoms, wherein the heteroatom is not adjacent to the N-Atom;

$R^3$,$R^4$ = independent from each x, y is hydrocarbyl, in particular alkyl, or halogen, wherein R is preferably substituted at the 2 or 6 position of the aryl, and further wherein R is branched at the 2-position;

x = independent from each $R^3$or $R^4$ 0 to 3.

Item 22: The process according to item 20 or 21 wherein the reaction is carried out in a hydrocarbon solvent in particular an aromatic solvent, preferably at temperatures of 30 to 100°C, in particular 40 to 90°C.

Item 23: The process according to one of items 20 to 22 wherein the Di-μ-halogen-bridged bis guanidinato tetrahalogen di zirconium compound, preferably as further defined under item 21, is obtained by precipitation, preferably by crystallisation.

Item 24: A process for the manufacture of a zirconium guanidinato alkyl compound comprising the following steps:

- bringing together
  a Di-μ-halogen-bridged bis guanidinato tetrahalogen di zirconium compound, preferably as further defined under item 21,
  with a Grignard-Reagent, wherein the Grignard-Reagent is preferably used in a 2.8 to 3.2 times molar excess relative to the Zr.

Item 25: The process of item 24 wherein independent form each other
the Di-μ-halogen-bridged bis guanidinato tetrahalogen di zirconium compound, preferably as further defined under item 21, is obtainable by the process of any of items 20 to 23
the Grignard-Reagent is Alkyl Mg Hal, wherein
Hal is independent from each other Halogen, in particular Cl;
Alkyl is C1 to C20 Alkyl, in particular Methyl or Ethlyl.

Item 26: The process of item 24 or 25 wherein the zirconium guanidinato alkyl compound is

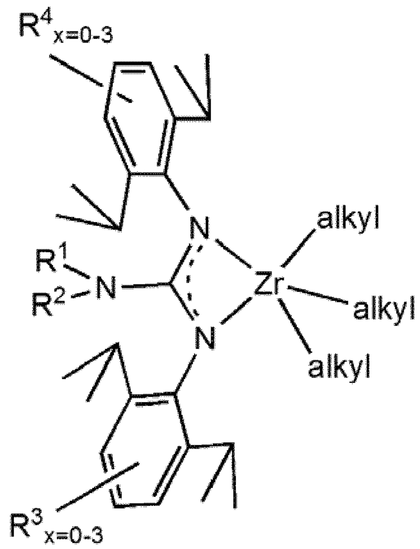

with

| | |
|---|---|
| $R^1$,$R^2$ | being C1 to C40 hydrocarbyl-, optionally comprising one or more heteroatoms, wherein the heteroatom is not adjacent to the N-Atom; |
| R $^3$,$R^4$= | independent from each x, y is hydrocarbyl, in particular alkyl, or halogen, wherein R is preferably substituted at the 2 or 6 position of the aryl, and further wherein R is branched at the 2-position; |
| x = | independent from each $R^3$or $R^4$ 0 to 3. |

Item 27: The process according to any one of items 24 to 26
wherein the reaction is carried out in a solvent and the solvent is a hydrocarbon, preferably a saturated C4- to C14-hydrocarbon and/or
wherein the zirconium guanidinato alkyl compound is obtained by precipitation, preferably by crystallisation.

Item 28: Use of the compound obtained according to the process of items 24 to 28 in the process of any one of items 1 to 19 as a CCTP catalyst.

DETAILED DESCRIPTION OF THE INVENTION

Hereinafter the components of the catalyst system applied and the growth composition are described in detail.

1 CCTP catalyst and their ligands

1.1 The coordinative chain transfer polymerisation (CCTP) catalyst comprises one transition metal compound selected from (according to IUPAC)

- group III, in particular scandium, yttrium, lanthanum, samarium and actinium;
- group IV, in particular Titanium or Zirconium, most preferably Zirconium;
- group V, in particular vanadium and niobium;
- group VI, in particular chromium,
- group VIII, in particular iron,
- group IX, in particular cobalt ;
- group X, in particular nickel, palladium and platinum

of the periodic table. Useful ligands, one or two per transition metal are selected from cyclopentadienyl, indenyl, fluorine, diamide ligands, phenoxy-imine-ligand, indolide-imine-ligands, amidinate, guanidinate, amidopyridine, pyridinimine and alcoholate each optionally substituted.

1.1.1 Group IV

Particularly, preferred metals are Ti, Zr or Hf in the +2, +3 or +4 formal oxidation state, preferably in the +4 formal oxidation state.

1.2 A particularly preferred ligand is a Guanidine-based metal-complex comprising one of the following ligands:

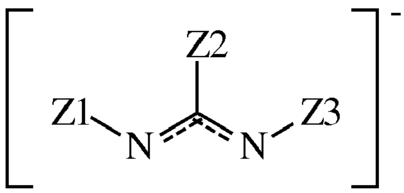

I

with
Z2 = NR1 R2,
R1 and R2 are independently from each other hydrocarbon moieties, in particular C1 to C40, preferably C1 to C18, optionally substituted hydrocarbon moieties additionally comprising (not directly adjacent to the N-Atom) one or more nitrogen, oxygen, and/or silicon atom(s), further optionally linked with each other or with Z1 and/or Z3.
Z1 and Z3 independently from each other are:

hydrocarbon moieties, in particular C1 to C40, preferably C3 to C22, most preferably C8 to C18 or more preferably C10 to C22, optionally linked with each other or with Z2, Z1 and Z3 optionally additionally comprising one or more nitrogen, oxygen, and/or silicon atom(s) (not directly adjacent to the N-Atom);
preferably alkyl in particular C1 to C40, preferably C3 to C22, most preferably C8 to C18, or aryl moieties, in particular C6 to C22, most preferably C8 to C18, optionally further substituted by hydrocarbyl groups, in particular C1 to C12, preferably C2 to C6, in particular alkyl, alkenyl or aryl groups, Z1 and Z3 optionally additionally comprising one or more nitrogen, oxygen and/or silicon atom(s) (not directly adjacent to the N-Atom); and
substituted phenyl, in particular tolyl, in particular substituted in the 2 and / or 6 position, mono- or di- or tri-isopropyl phenyl, in particular 2,6-diisopropyl phenyl, mono- or di- or tri-t-butyl phenyl, in particular 2,6 di-t-butyl phenyl, mono- or di- or tri-(C1 to C4)alkoxy phenyl, in particular 2,6-di- (C1 to C4)alkoxy phenyl, or mono- or or di-(C1 to C4)alkylamino phenyl, in particular 2,6-di-(C1 to C4) alkylamino phenyl.

Z1 and Z3 each comprise more carbon atoms than Z2, for example Z1 and Z3 each comprise 8 carbon atoms and more. Most preferably and independent of the above Z1 and Z3 are branched or substituted in one or more of the 2-positions.

1.3 The metal complexes preferably have the following structure

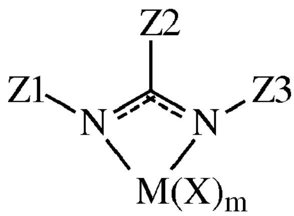

II

wherein
M = Ti, Zr or Hf, preferably Ti or Zr, more preferably Zr
X = independent of each m halogen, preferably Cl; hydrocarbyl, in particular C1 to C40, preferably C1 to C4, in particular methyl; hydride; alkoxide; amide, optionally substituted, NR1 R2 with R1 and R2 as defined above, preferably NR1 R2 is di-ethylamido, dimethylamido or methylethylamido; tetrahydrofuran; m = 1 to 4,
with Z1, Z2 and Z3 as defined above.

Most preferably the metal complex has the following structure:

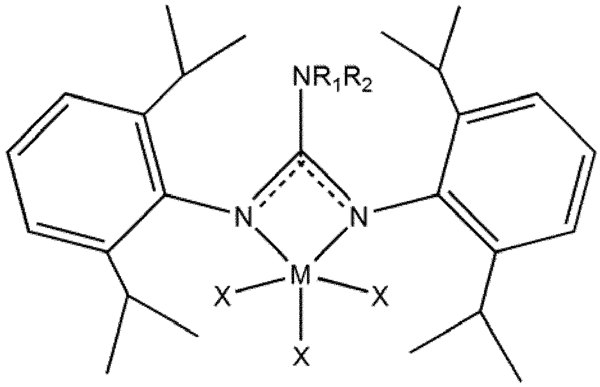

III

wherein
M = Ti, Zr, preferably Zr
X = halogene, preferably Cl, more preferably hydrocarbyl, in particular methyl, preferably NR1 R2 is diethylamido, dimethylamido or methylethylamido

The above mentioned complexes as defined by structures III may also exist as anionic species with an additional cation $Q^+$ which for example is selected from the group of $R_4N^+$, $R_3NH^+$, $R_2NH_2^+$, $RNH_3^+$, $NH_4^+$, $R_4P^+$ in which R is an alkyl, aryl, phenyl, hydrogen or halogen.

Examples of the above metal catalysts include
{N',N"-bis[2,6-di(isopropyl)phenyl]-N,N-dimethyl-guanidinato}metal(IV) chloride,
{N',N"-bis[2,6-di(isopropyl)phenyl]-N,N-diethyl-guanidinato}metal(IV) chloride,
{N',N"-bis[2,6-di(isopropyl)phenyl]-N,N-pentamethylene-guanidinato} metal (IV) chloride,
{N',N"-bis[2,6-di(isopropyl)phenyl]-N-cyclohexyl-N-methyl-guanidinato} metal (IV) chloride,
{N',N"-bis[2,6-di(isopropyl)phenyl]-N-cyclohexyl-N-methyl-guanidinato} metal (IV) chloride,
[Diethylammonium][N,N'-bis(2,6-diisopropylphenyl)-benzamidinato-tetrachloro]metalat(IV),
[Diethylammonium][N,N'-bis(2,6-diisopropylphenyl)-4-(dimethylamino)benzamidinato-tetrachloro]metalat(IV),
[Diethylammonium][N,N'-bis(2,6-diisopropylphenyl)-4-methoxybenzamidinato-tetrachloro]metalat(IV),
[Diethylammonium][N,N'-bis(2,6-diisopropylphenyl)-4-(2,5-dimethyl-1 H-pyrrol-1-yl)benzamidinato-tetrachloro]metalat(IV),
[N,N'-bis(2,6-diisopropylphenyl)-4-(dimethylamino)benzamidinato-diethylamido]trimethylmetal(IV),
[N,N'-bis(2,6-diisopropylphenyl)-4-(2,5-dimethyl-1 H-pyrrol-1-yl)benzamidinato-diethylamido] trimethylmetal(IV),
{N',N"-bis[2,6-di(isopropyl)phenyl]-N,N-dimethyl-guanidinato}trimethylmetal(IV),
{N',N"-bis[2,6-di(isopropyl)phenyl]-N,N-diethyl-guanidinato}trimethylmetal(IV),
{N',N"-bis[2,6-di(isopropyl)phenyl]-N,N-pentamethylene-guanidinato}trimethylmetal (IV),
{N',N"-bis[2,6-di(isopropyl)phenyl]-N-cyclohexyl-N-methyl-guanidinato} trimethylmetal (IV),
{N',N"-bis[2,6-di(isopropyl)phenyl]-N-cyclohexyl-N-methyl-guanidinato} trimethylmetal (IV) chloride,
preferably with metal = titan or zirconium.

Alternatively, the metal complex may be formed in situ from suitable transition metal and ligand precursors. The structure of the resulting in situ complex is as defined for the complexes (structure II and III) above.

The transition metal precursor may be any Ti, Zr or Hf complex capable of reacting with a ligand precursor to form a guanidinate complex as described above in situ.

Examples of such transition metal precursor (with M = Ti, Zr or Hf) include:
$MX_4$ where each X may independently halogen {F, Cl, Br, I}, hydride {H}, hydrocarbyl {R, *e.g.* benzyl}, alkoxide {OR} or amide {NR1R2});
$MX_4L_2$ where each X may independently halogen {F, Cl, Br, I}, hydride {H}, hydrocarbyl {R, e.g. benzyl}, alkoxide {OR} or amide {NR1R2} with L equals any two electron donor ligand, e.g. ethers such as tetrahydrofuran,or diethylether, acetonitrile, or trihydrocarbylphosphine;
$M(acac)_4$, where acac = 2,4-pentanedionato, 1,1,1,5,5,5-hexafluoro-2,4-pentanedionato or 2,2,6,6-tetramethyl-3,5-heptanedionato; $M(O_2CR)_4$, where $O_2CR$ is any carboxylic acid anion, e.g. 2-ethylhexanoate.

The ligand precursor may be any compound capable of reacting with a transition metal precursor to form an amidine or guanidine complex in situ. Examples of such ligand precursor include:

dihydrocarbylcarbodiimides, such as bis(2,6-diisopropylphenyl)carbodiimide or dicyclohexylcarbodiimide,
diheterohydrocarbylcarbodiimides, such as bis(2-methoxyphenyl)-carbodiimide;
amidate or guanidate salts, e.g. lithium 1,3-dihydrocarbylamidate or lithium 1,3-dihydrocarbylguanidate; or

guanidines, such as 2,3-bis(2,6-diisopropylphenyl)-1,1-dihydrocarbylguanidine

1.4. The metal complexes become a catalyst for CCTP when combined at least with a co-catalyst.

The co-catalyst, without being bound to the theory, acts as a chain transfer agent and may optionally act in addition as an activator for the complex in order that the complex becomes the (active) catalyst.

2.0 Activator

The activator may comprise a boron containing compound such as a borate. More preferably the activator comprises pentafluorophenyl boranes and pentafluorophenyl borates. Illustrative examples of boron compounds which may be used as activator in the preparation of catalysts of this invention are tri-substituted (alkyl) ammonium salts such as trimethylammonium tetraphenylborate, triethylammonium tetraphenylborate, tripropylammonium tetraphenylborate, tri(n-butyl)ammonium tetraphenylborate, tri(t-butyl)ammonium tetraphenylborate, N,N-dimethylanilinium tetraphenylborate, N,N-diethylanilinium tetraphenylborate, N,N-dimethyl-2,4,6-trimethylanilinium tetraphenylborate, trimethylammonium tetrakis(pentafluorophenyl) borate, triethyl-ammonium tetrakis(pentafluorophenyl) borate, tripropylammonium tetrakis(pentafluorophenyl) borate, tri(n-butyl)ammonium tetrakis(pentafluorophenyl) borate, tri(sec-butyl)ammonium tetrakis(pentafluorophenyl) borate, dioctadecylmethylammonium tetrakis(pentafluorophenyl)borate, dioctadecylmethylammonium tetrakis-(3,5-bis(trifluoromethyl)-phenyl)borate, N,N-dimethylanilinium tetrakis(pentafluorophenyl) borate, N,N-dimethylanilinium n-butyltris(pentafluorophenyl) borate, N,N-dimethylanilinium benzyltris(pentafluorophenyl) borate, N,N-dimethylanilinium tetrakis(4-(t-butyldiimethylsilyl)-2,3,5,6-tetrafluorophenyl) borate, N,N-dimethyl-anilinium tetrakis(4-(triisopropylsilyl)-2,3,5,6-tetrafluorophenyl) borate, N,N-dimethylanilinium pentafluorophenoxytris(pentafluorophenyl) borate, N,N-diethylanilinium tetrakis(pentafluorophenyl) borate, N,N-dimethyl-2,4,6-trimethyl-anilinium tetrakis(pentafluorophenyl) borate, trimethylammonium tetrakis(2,3,4,6-tetrafluorophenyl) borate, triethylammonium tetrakis(2,3,4,6-tetrafluorophenyl) borate, tripropylammonium tetrakis(2,3,4,6-tetrafluorophenyl) borate, tri(n-butyl)-ammonium tetrakis(2,3,4,6-tetrafluorophenyl) borate, dimethyl(t-butyl )ammonium tetrakis(2,3,4,6-tetrafluorophenyl) borate, N,N-dimethylanilinium tetrakis(2,3,4,6-tetrafluorophenyl) borate, N,N-diethylanilinium tetrakis(2,3,4,6-tetrafluorophenyl) borate, and N,N-dimethyl-2,4,6-trimethylanilinium tetrakis(2,3,4,6-tetrafluorophenyl) borate;
dialkyl ammonium salts such as: di-(i-propyl)ammonium tetrakis(pentafluorophenyl) borate, and dicyclohexylammonium tetrakis(pentafluorophenyl) borate; tri-substituted phosphonium salts such as: triphenylphosphonium tetrakis(pentafluorophenyl) borate, tri(o-tolyl)phosphonium tetrakis(pentafluorophenyl) borate, and tri(2,6-dimethylphenyl)phosphonium tetrakis(pentafluorophenyl) borate;
di-substituted oxonium salts such as: diphenyloxonium tetrakis(pentafluorophenyl) borate, di(o-tolyl)oxonium tetrakis(pentafluorophenyl) borate, and di(2,6-dimethylphenyl)oxonium tetrakis(pentafluorophenyl) borate;
di-substituted sulfonium salts such as: diphenylsulfonium tetrakis(pentafluorophenyl) borate, di(o-tolyl)sulfonium tetrakis(pentafluorophenyl) borate, and bis(2,6-dimethylphenyl)sulfonium tetrakis(pentafluorophenyl) borate.

The activator may alternatively comprise an aluminium containing compound such as an aluminate. More preferably the activator comprises an tetrakisalkyloxy-aluminate or tetrakisaryloxy-aluminate, in particular Tetrakis-C1 to C6-alkyloxy-aluminate or tetrakisaryloxy-aluminate, the alky or aryl being $-CF_3$ substituted, such as $[Al(OC(Ph)(CF_3)_2)_4]^-$ or $[Al(OC(CF_3)_3)_4]^-$.

3.0 Chain Shuttling Agent CSA (=co-catalyst)

The CSA a chain shuttling agent (CSA) being one or more metal alkyls selected from the group II, XII and XIII from the periodic table, The CSA preferably is a C1 to C30 hydrocarbyl metal compound, methylaluminoxane or both, the metal being aluminium, zinc, magnesium, indium or gallium, preferably trihydrocarbyl aluminium, dihydrocarbyl magnesium or dihydrocarbyl zink.

According to one embodiment of the invention it is preferred to use a mixture in particular a mixture of tri C1- to C3- alkyl aluminium and di- C1- to C3- alkyl zinc.

Most preferably the CSA (co-catalyst) is selected from:

tri hydrocarbyl aluminium, wherein the hydrocarbyl is for example methyl, ethyl, propyl, isopropyl, n-butyl, isobutyl, pentyl, neopentyl or isopentyl or a mixtures thereof, preferably tri(methyl and/or ethyl) aluminium,
di-hydrocarbyl zinc, wherein the hydrocarbyl is for example methyl, ethyl, propyl, isopropyl, n-butyl, isobutyl, pentyl, neopentyl or isopentyl or a mixtures thereof, preferably di(methyl and/or ethyl) zinc,
a mixture of tri hydrocarbyl aluminium and di-hydrocarbyl zinc reagents as described above,
oligomeric or polymeric hydrocarbyl alumoxanes, preferably oligomeric or polymeric methyl alumoxanes (including modified methylalumoxane, modified by reaction of methylalumoxane with triisobutyl aluminium or isobutylalumoxane),

hydrocarbyl aluminium halogenides such as dialkyl aluminium halogenides, alkyl aluminium dihalogenides, with alkyl preferably being C1 to C3-alkly,
hydrocarbyl aluminium sesqui halogenides, preferably. methyl aluminium sesqui halogenides,
di-hydrocarbyl magnesium, wherein the hydrocarbyl is for example methyl, ethyl, propyl, isopropyl, n-butyl, isobutyl, pentyl, neopentyl or isopentyl or a mixtures thereof, preferably di(methyl and/or ethyl and/or butyl) magnesium;
tri-hydrocarbyl indium, wherein the hydrocarbyl is for example methyl, ethyl, propyl, isopropyl, n-butyl, isobutyl, pentyl, neopentyl or isopentyl or a mixtures thereof, preferably tri(methyl and/or ethyl and/or butyl) indium;
tri-hydrocarbyl gallium, wherein the hydrocarbyl is for example methyl, ethyl, propyl, isopropyl, n-butyl, isobutyl, pentyl, neopentyl or isopentyl or a mixtures thereof, preferably tri(methyl and/or ethyl and/or butyl) gallium or mixture thereof.

The most preferred CSA (acting also as co-catalyst) for use in forming the (active) catalysts is triethylaluminium or a mixture of triethylaluminium comprising minor portions of diethylaluminiumhydrid (such as below 10 wt.%).

4.0 Activated CCTP catalyst

The active CCTP catalysts are rendered catalytically active by combination of CCTP catalyst (see 1.0 CCTP catalyst and ligands) with a) an activating co-catalyst (CSA) (on its own) or b) by a combination of a co-catalyst (CSA) with an activator as listed under 2.0 activator.

In addition to above mentioned co-catalysts an activator can be used or is preferably to be used when the co-catalyst on its own is not activating. If the respective co-catalyst is selected from the trialkyl aluminium compounds use of activator is preferable. Suitable activators are referenced above.

The foregoing co-catalysts and activating techniques have been previously taught with respect to different metal complexes in the following references: EP 277003, US 5153157, US 5064802, EP 468651 and EP 520732, the teachings of which are hereby incorporated by reference.

The molar ratio of catalyst (CCTP catalyst) to co-catalyst (CSA) with reference to the [metal catalyst] to [CSA] atomic ratio preferably is from 1:1 to 1:10000000, more preferably 1:100 to 1:100000 and most preferably 1:1000 to 1:40000.

5.0 Chain displacement Catalyst (CDC)

The chain displacement catalyst is a Nickel or Cobalt compound. Typical compounds are Nickel and Cobalt compounds with one or more of the following substituent: halides, carbonyls, acetylacetonato, cyclooocta-1,5-diene, cyclopentadienyl, C1- to C12- octanoates, tri(C1- to C12- hydrocarbyl)-phosphines.

Most preferred are bis(cyclooctadienyl)nickel(0) and nickel(II) acetylacetonate.

6.0 Carrier

A support, especially silica, alumina, magnesium chloride, or a polymer (especially poly(tetrafluoroethylene or a polyolefin) may also be applied. The support is preferably used in an amount to provide a weight ratio of catalyst (based on metal): support from 1:100000 to 1:10, more preferably from 1:50000 to 1:20, and most preferably from 1:10000 to 1:30.

7.0 Solvent

Suitable solvents for polymerisation are preferably inert liquids. Suitable solvents include aliphatic and aromatic hydrocarbons, particularly C4 to C20 hydrocarbons or olefins, linear and/or branched, and mixtures thereof (including monomers subject to polymerisation, especially the previously mentioned addition polymerisable monomers and produced oligomerised olefins); cyclic and alicyclic hydrocarbons such as cyclohexane, cycloheptane, methylcyclohexane, methylcycloheptane, and mixtures thereof; isooctanes, aromatic and hydrocarbyl-substituted aromatic compounds such as benzene, toluene, and xylene, Mixtures of the foregoing are also suitable. Most preferred is toluene.

8.0 Olefins

In accordance with this invention C1- to C8-olefins, particularly alpha olefins, especially ethylene or ethylene and propylene or propylene are converted to oligomeric mono-unsaturated hydrocarbons, in short herein called oligomerised olefins.

9.0 Process (conditions applicable to all modes of operation)

The Process for the manufacture of oligomerised olefins comprises
bringing in contact with each other at least

(a) the one or more olefins as defined above,
(b) the coordinative chain transfer polymerisation catalyst (CCTP) as defined above,
(c) the chain shuttling agent (CSA) as defined above.

(a), (b) and (c) form the obligatory components of the growth composition, preferably in one reactor.

The growth composition contains further before or during the oligomerisation the chain displacement catalyst (CDC) according to one embodiment (simultaneous process) and according to another embodiment the chain displacement catalyst (CDC) is added when the oligomerisation is substantially finished and the CCTP is inactivated (sequential process).

The products obtained are the oligomerised olefins described herein below.

The order of bringing the components together is not of particular relevance. Nevertheless typically a solvent is provided first and the solvent is saturated with the olefin.

Suspension, solution, slurry, gas phase, solid state powder oligomerisation or other process condition may be applied as desired.

In general, the oligomerisation may be accomplished at temperatures from 20 to 200 °C, preferably 50 to 100 °C, most preferably 60-90 °C, and pressures from 1 to 100 bar, preferably 1 to 30 bar. In general, shorter olefins can be produced if the reaction temperature is increased and pressure is decreased. The distribution can be shifted from Schulz-Flory to Poisson via the applied CCTP catalyst system, the CSA, the activator and displacement catalyst. The distribution can additionally be tuned by the catalyst/CSA/CDC ratio. The distribution can also be altered via temperature and applied pressure. The produced olefins can be purified via mechanical or thermal purification processes. In general filtration and distillation can be applied for purification purposes.

In the process for obtaining an oligomerised olefin by applying CCTP and subsequently the CDC, the olefin is obtained in a Poisson distribution, wherein the molar ratio of the coordinative chain transfer polymerisation catalyst (CCTP catalyst) to the chain shuttling agent (CSA) is 1 : > 10000, preferably 1 : > 100000. The chain length can be tuned by the amount of olefin oligomerised.

It was found that higher concentrations or an increase of the (partial) pressures of the C2- to C8- olefin results in a higher oligomerisation degrees. Higher means above 4 bar.

It was further found that a higher reaction temperature results in a lower oligomerisation degree.

The process according to the invention can be carried out in two different modes:

a) via a simultaneous process where CCTP catalyst, CSA and CDC (and optionally activator) are present at the same time, e.g. from the start; or
b) via a sequential process where at first CCTP catalyst, and CSA (and optionally activator) are present but not CDC und at a later stage the CCTP is deactivated and the CDC is added.

9.1 Simultaneous Process

If in the regular reaction mode CCTP and CDC both are present in the reaction composition, chain growth and chain displacement take place at the same time and high ratios of CSA to CCTP and of CDC to CCTP result in short chain lengths with a Schulz-Flory distribution.

However, if in the regular reaction mode low CSA concentrations (CCTP / CSA 1 : < 1000, in particular 1: 100 to 1: 500) and low CDC concentrations (CCTP / CDC 1 : 1 to 1: 2) are applied, mainly a Poisson distribution is obtained. In general the product distribution can either be tuned by the applied type of CCTP, CSA and CDC and by the ratio of CCTP/CSA/CDC or by the applied reaction conditions, mainly pressure and temperature. Increasing ethylene pressure results in higher molecular weight olefins and broader distribution, while increasing temperature yields more short chain olefins with a more narrow distribution.

However, the biggest influence on the type of distribution of chain lengths obtained has the applied CCTP catalyst system. For instance a CCTP catalyst with a higher transfer to propagation rate ($K_t \geq K_p$, Scheme 2), e.g. guanidinato zirconium complexes, yields at equivalent reaction conditions mainly a Schulz-Flory distribution of alpha-olefins, while a CCTP catalyst with $K_t \leq K_p$, e.g. guanidinato titanium catalysts, gives mainly Poisson distribution one.

When applying the simultaneous reaction mode CCTP and CDC (as well as CSA) are present during the whole reaction typically resulting in oligomerised olefins, wherein more that 80 wt% of olefins are obtained in a Schulz-Flory distribution, wherein the molar ratio of the coordinative chain transfer polymerisation catalyst (CCTP catalyst) to the

chain shuttling agent (CSA) is 1 : < 10000, preferably 1 : < 1000; and the molar ratio of the coordinative chain transfer polymerisation catalyst (CCTP catalyst) to the chain displacement catalyst (CDC) is 1 : > 2, preferably 1 : 5 to 1 : 10.

According to a different way of conducting the simultaneous reaction mode the oligomerised olefins are being obtained in a mainly Poisson distribution, wherein the molar ratio of the coordinative chain transfer polymerisation catalyst (CCTP catalyst) to the chain shuttling agent (CSA) is 1 : < 10000, preferably 1 : < 1000; and the molar ratio of the coordinative chain transfer polymerisation catalyst (CCTP catalyst) to the chain displacement catalyst (CDC) is 1 : < 10, preferably 1 : < 4.

The olefin is further preferably obtained in a Schulz-Flory distribution, if the molar ratio of the coordinative chain transfer polymerisation catalyst (CCTP catalyst) to the chain shuttling agent (CSA) is 1 : > 1000, preferably 1 : > 10000; and the molar ratio of the coordinative chain transfer polymerisation catalyst (CCTP catalyst) to the chain displacement catalyst (CDC) is 1 : > 10, preferably 1 : 10 to 1 : 20.

If the process is carried out with a C2 to C3 olefin and a pressure of lower than 4 bar, preferably lower than 2 bar, the oligomerised olefin is being obtained predominately in a Schulz-Flory distribution.

The process can be performed in one single reactor without any intermediate steps or transfers.

9.2 Sequential Process

According to one embodiment of the invention comprising a sequential reaction the CDC is subsequently brought in contact with the reaction composition comprising the inactivated CCTP catalyst, the CSA and the oligomerised olefin, the reaction composition not comprising the CDC, wherein the CCTP catalyst is inactivated by heating the reaction composition, most preferably above 120°C or adding catalysts poisons, the catalyst poisons being preferably selected from the group consisting of halogenated metal alkyls alkali and earth alkali salts, the catalysts poisons being selected in a manner that the CCTP catalyst is inactivated but not the CSA and not the CDC to be added.

For the sequential reaction mode it is preferred to use a molar ratio of the coordinative chain transfer polymerisation catalyst (CCTP catalyst) to the chain displacement catalyst (CDC) of 1 : 0.05 to 1 : 100, preferably 1 : 1 to 1 : 2. Optionally the molar ratio of the coordinative chain transfer polymerisation catalyst (CCTP catalyst) to the chain shuttling agent (CSA) is preferably 1 : > 50000. For the sequential reaction mode the CDC catalyst is preferably added at a temperature above 120°C.

The sequential reaction mode results in a Schulz-Flory distribution, of the oligomerised olefins only at low molar ratios of olefin to CSA. Otherwise the sequential reaction mode results in a Poisson distribution, in particular if the C2 to C3 pressure is greater than 2 bar. In other words in case of sub-sequent addition of CDC and at CSA conversion above 20% increasing amounts of CSA give shorter chain length with a mainly Poisson distribution, below 20% conversion the process will result in a product with a mainly Schulz-Flory distribution.

10.0 Product

Desirably the oligomerisation is conducted by contacting the monomer(s) and catalyst composition under conditions to produce an oligomer or a polymer having molecular weight (MW [g/mol]) from 56 to 1000000, preferably 56 to 10000, most preferably 84 to 1000.

In particular it may be wanted that high molecular oligomers (>1000 g/mol) are produced. For determination of the molecular weight distribution gel permeation chromatography (GPC) or mass spectroscopy may be used. For olefins having molecular weight below 1000 standard gas chromatography can be applied.

GPC-samples were prepared by dissolving the polymer (0.05 wt.-%, conc. = 1 mg/mL) in the mobile phase solvent in an external oven and were run without filtration. The molecular weight was referenced to polyethylene (Mw = 520 to 3200000 $gmol^{-1}$) and polystyrene (Mw = 580 to 2800000 $gmol^{-1}$).

The distribution of the chain lengths of the olefins obtained can be influenced as follows:

Simultaneous Process

| CCTP | CSA | CDC | CCTP:CSA | CCTP:CDC | Dominant distribution of products |
|---|---|---|---|---|---|
| IV (Zr) | TEAL | Ni | 1:<1000 | 1:<4 | POISSON |
| IV (Zr) | TEAL | Ni | 1:<1000 | 1:5-1:10 | Schulz-Flory |
| IV (Zr) | TEAL | Ni | 1:>10000 | 1:10-1:20 | Schulz-Flory |
| I (Ti) | TEAL | Ni | 1:>10000 | 1:<20 | POISSON |

Sequential Process

| CCTP | CSA | CDC | CSA conversion | Dominant distribution of products |
|---|---|---|---|---|
| IV (Zr) | TEAL | Ni | >20% | POISSON |
| IV (Zr) | TEAL | Ni | <20% | Schulz-Flory |
| I (Ti) | TEAL | Ni | 10-100% | POISSON |

11. Synthesis of preferred CCTP Catalysts

In the preparation of the CCTP catalyst highly selective synthesis routes are highly preferred as intermediate compounds and by products also exhibit polymerisation activity often yielding undesired molecular weight olefins. The most preferred catalysts according to this invention are complexes IV which are obtained with high selectivity by reacting $Zr(NEt_2)Cl_3(Et_2O)$ with Ar-NCN-Ar in a first step to obtain III and reacting III in a second step with 6 moles of methyl magnesium chloride in hexane.

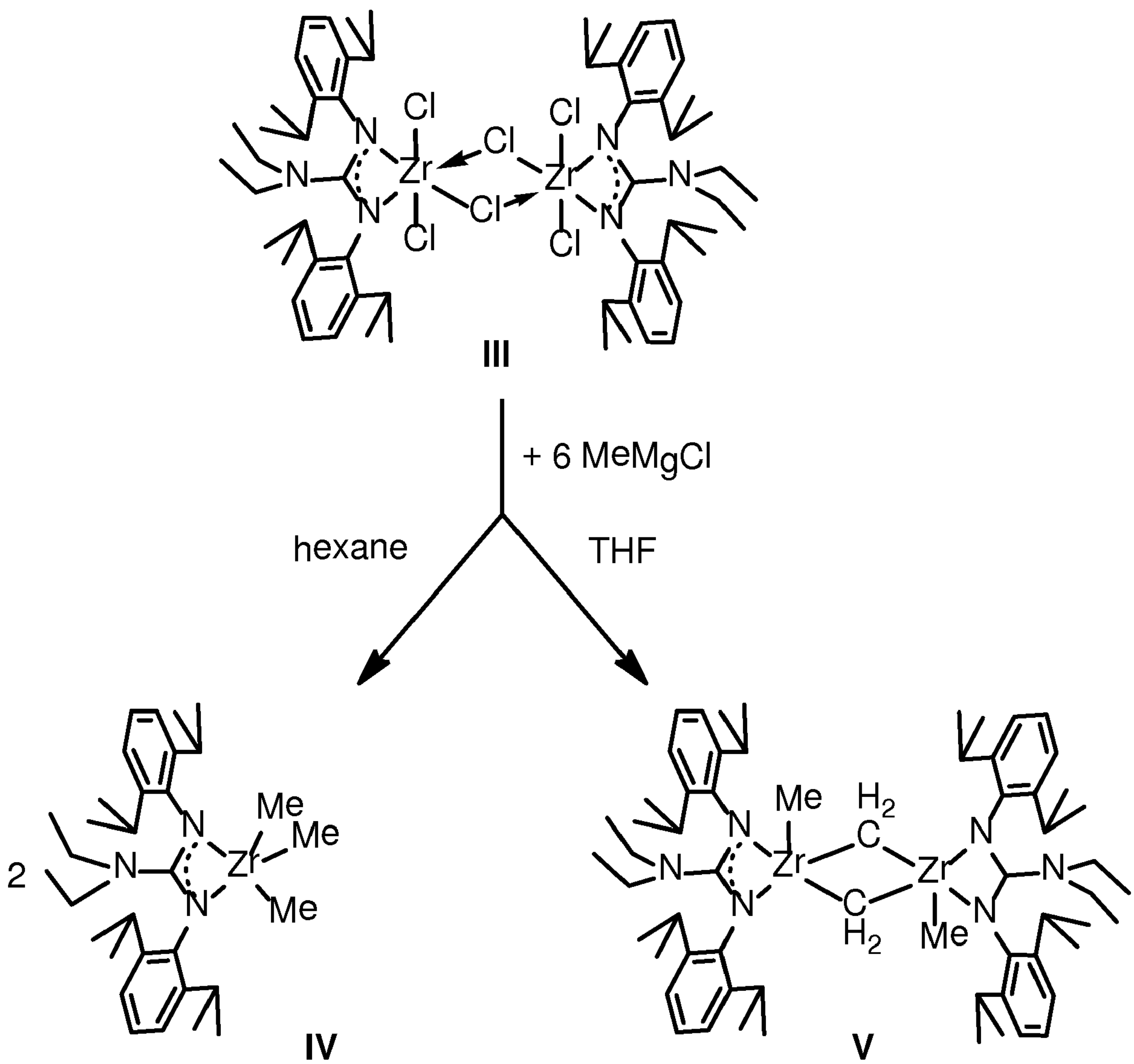


III

IV

V

The same reaction in THF yields the methylene bridged dimer di-μ-methylene-bis[2,3-bis(2,6-diisopropylphenyl)-1,1-diethylguanidinato]-dimethanido-dizirconium(IV), V μ-$CH_2$-{$[Et_2NC(2,6\text{-}Pr^i{}_2C_6H_3N)_2]ZrMe_2$}$_2$ instead, which is much less active and less selective. Reacting I with 3 equivalents of Methyl Grignard reagent did not yield the expected trimethyl analog but the less preferred {*N',N''*-bis[2,6-di(isopropyl)phenyl]-*N,N*-dialkyl-guanidinato}-(diethylamido)-dimethanido-zirconium(VI) complexes, II {$[RR'NC(2,6\text{-}Pr^i{}_2C_6H_3N)_2](Et_2N)ZrMe_2$, instead.

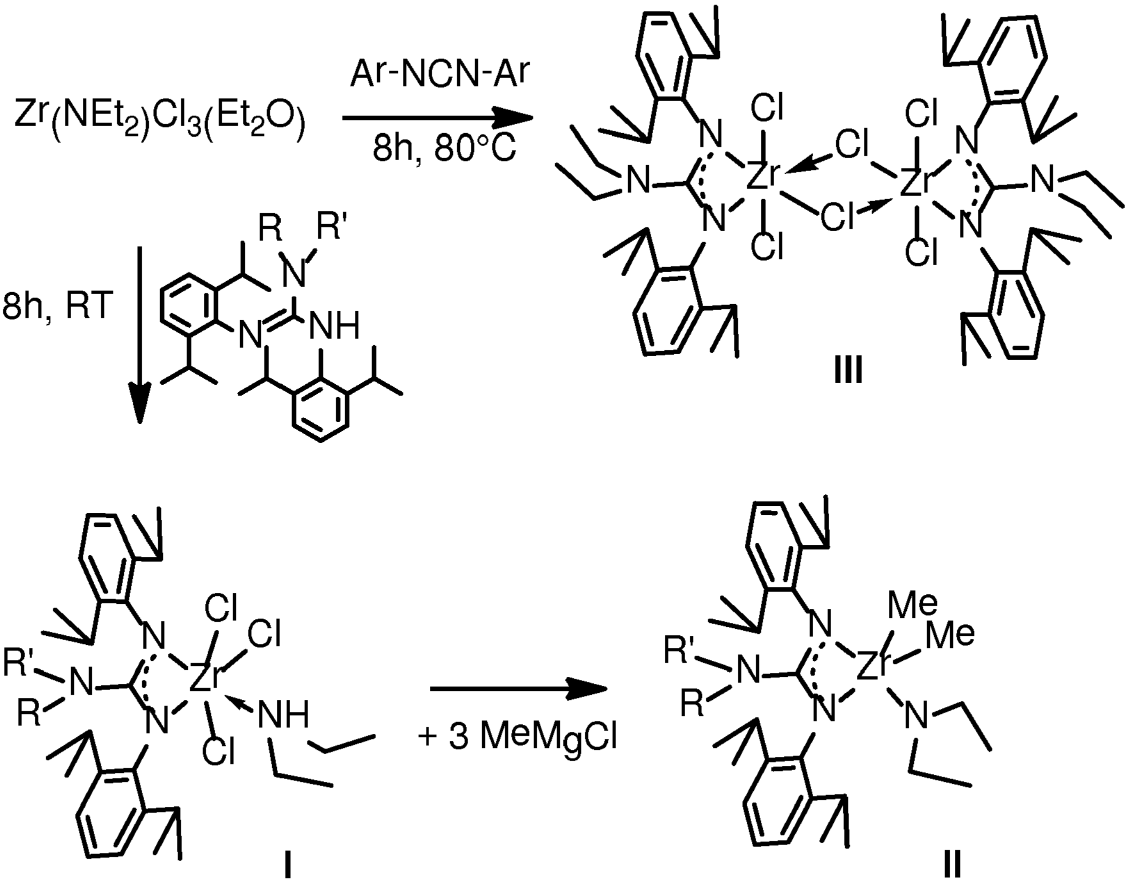


| | a | b | c | d |
|---|---|---|---|---|
| R = | Et | Me | Me | 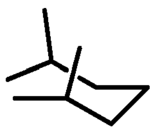 |
| R' = | Et | Ph | Cy | |

Ar = 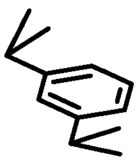

The new well-defined catalyst (structure IV) can therefore improve the earlier described CCTP process by reducing the high molecular weight polymer side products by simultaneously enhancing the catalyst activity.

**Figures**

The following is depicted in the attached figures:

Fig. 1: Reaction scheme illustrating the assumed mechanism of the tandem catalyst oligomerisation process of ethylene

Fig. 2: Molecular structure of III in the formula as displayed above.

Fig. 3: Molecular structure of IV as described in the formula as displayed above.

Fig. 4: 1H NMR spectrum ($C_2Cl_4D_2$, 120°C) of oligomers obtained with $GuaTiMe_3$ (I) precatalyst in absence (entry 1, table 1, above) and presence of $Ni(COD)_2$ (entry 5, table 2, below). In the absence of a CDC no olefins are observed. With $Ni(COD)_2$ only alpha-olefins were observed.

Fig.5: 1H NMR spectrum ($C_2Cl_4D_2$, 120°C) of oligomers obtained with $GuaZrMe_3$ (III) precatalyst in absence (entry 2, below, table 1) and presence of $Ni(COD)_2$ (entry 9, above, table 2). In absence of a CDC no olefins are observed.

Fig. 6: 1H NMR spectrum ($C_2Cl_4D_2$, 120°C) of oligomers obtained with GuaZrMe3 (III) precatalyst in presence of $Ni(COD)_2$ (entry 9, below, table 2), $Ni(acac)_2$ (entry 6, middle, table 2) and $Ni(stea)_2$ (entry 7, above, table 2). From top to the bottom the ratio between terminal to internal olefins increases.

Fig.7: Linear alpha-olefin distribution at different ethylene pressure; 8 μmol $GuaZrMe_3$, 16 μmol Dimethylanilinium-borat, 32 μmol $Ni(COD)_2$, 8000 μmol TEAL, T = 60 °C

Fig. 8: Linear alpha-olefin distribution at different temperatures; 8 μmol GuaZrMe3, 16 μmol Dimethylaniliniumborat, 32 μmol Ni(COD)2, 8000 μmol TEAL, p = 2 bar.

Figure 9 Linear alpha-olefin distribution at different temperatures; 4 μmol GuaZrMe3, 4 μmol Trioctyammonium borate, 8 μmol Ni(COD)2, 40000 μmol TEAL, p = 2 bar, 22g ethylene.

## Experimental Section

The following abbreviations were used:

| | |
|---|---|
| Me | - Methyl ($CH_3$) |
| Et | - Ethyl ($CH_3CH_2$) |
| TEAL | - Triethyl aluminium ($Et_3Al$) |
| GuaH | - 2,3-bis(2,6-diisopropylphenyl)-1,1-diethylguanidine |
| i-Pr | - *iso*-Propyl ($Me_2CH$) |
| i-Bu | - *iso*-Butyl ($Me_2CHCH_2$) |
| Cy | - Cyclohexyl ($C_6H_{11}$) |
| TMA | - Trimethylaluminium ($Me_3Al$) |
| TEA | - Triethylaluminium ($Et_3Al$) |
| DEAC | - Diethylaluminiumchloride [$(Et_2AlCl)_2$] |
| Pipi | - cis-2,6-Dimethylpiperidin-1-yl [*cis*-2,6-$Me_2C_5H_8N$] |
| COD | - Cyclooctadiene ($C_8H_{12}$) |
| acac | - Acetylacetonate ($CH_3COCHCOCH_3^-$) |
| stea | - Stearate ($O_2C(CH_2)_{16}CH_3^-$) |
| $Ni(acac)_2$ | - Nickel(II) acetylacetonate ($Ni(C_5H_7O_2)_2$) |
| $Ni(COD)_2$ | - Bis(1,5-cyclooctadiene)nickel(0) ($Ni(C_8H_{12})_2$) |
| $Ni(stea)_2$ | - Nickel(II) stearate ($Ni(O_2C(CH_2)_{16}CH_3$ |

All ratios herein are molar ratios except when specifically mentioned otherwise.

General: All manipulations of air- or moisture-sensitive compounds were carried out under $N_2$ using glove-box, standard Schlenk, or vacuum-line techniques. Solvents and reagents were purified by distillation from $LiAlH_4$, potassium, Na/K alloy, or sodium ketyl of benzophenone under nitrogen immediately before use. Toluene (Aldrich, anhydrous, 99.8%) was passed over columns of $Al_2O_3$ (Fisher Scientific), BASF R3-11 supported Cu oxygen scavenger, and molecular sieves (Aldrich, 4 Å). Ethylene and Propylene (AGA polymer grade) were passed over BASF R3-11 supported Cu oxygen scavenger and molecular sieves (Aldrich, 4 Å). NMR spectra were recorded on a Varian Inova 400 ($^1$H: 400 MHz, $^{13}$C: 100.5 MHz) or Varian Inova 300 (1 H: 300 MHz, 13C: 75.4 MHz) spectrometer. The $^1$H and $^{13}$C NMR spectra, measured at 26°C, were referenced internally using the residual solvent resonances, and the chemical shifts (δ) reported in ppm. High temperature NMR measurements of polymer samples were carried out in deutero tetrachloethane at 120°C.

Gel permeation chromatography (GPC) analysis was carried out on a PL-GPC 220 (Agilent, Polymer Laboratories) high temperature chromatographic unit equipped with LS, DP and RI detectors and three linear mixed bed columns (Olexis, 13-micron particle size) at 150 °C using 1,2,4-trichlorobenzene as the mobile phase. The samples were prepared by dissolving the polymer (0.05 wt.-%, conc. = 1 mg/mL) in the mobile phase solvent in an external oven and were run without filtration. The molecular weight was referenced to polyethylene (Mw = 520 - 3200000 $gmol^{-1}$) and polystyrene (Mw = 580-2800000 $gmol^{-1}$) standards. The reported values are the average of at least two independent determinations.

GC analysis was performed with an Agilent 6850 gas chromatograph and/or Agilent 7890A GC with an inert MSD 5975C with Triple Axis Detector. Both GC's are equipped with an Agilent 19095J-323E capillary column (HP-5; 5 % phenyl methyl siloxane; 30 m; film 1.5 μm, diameter 0.53 mm) and a flame ionization detector.

N,N-Dimethylanilinium (tetrapentafluorophenyl) borate ($[PhNMe_2H][B(C_6F_5)_4]$), Nickel(II) stearate, Bis(1,5-cyclooctadiene)nickel(0), Nickel(II) pentanedionate (anhydrous; 95%), Titanium(IV)chloride and Zirconium(IV)chloride are commercially available from abcr GmbH & Co. KG. Triethyl aluminium (SASOL Germany GmbH) and Bis(2,6-diisopropylphenyl)carbodiimide (TCI Deutschland GmbH) were used as received. The ligand precursor 2,3-bis(2,6-diisopropylphenyl)-1,1-diethylguanidine (G. Jin, C. Jones, P. C. Junk, K.-A. Lippert, R. P. Rose, A. Stasch, New J. Chem, 2008, 33, 64-75) and the metal precursors diethylaminotri-chloridozirconium(IV) etherate (E. V. Avtomonov, K. A. Rufanov, Z. Naturforsch. 1999, 54 b, 1563-1567) and 2,3-Bis(2,6-diisopropylphenyl)-1,1-diethylguanidinato trimethanido titanium(IV) ($GuaTiMe_3$, I, J. Obenauf, W. P. Kretschmer, R. Kempe, Eur. J. Inorg. Chem. 2014, 1446-1453) were prepared according to published procedures.

Comparative Example 1: Synthesis of {2,3-bis[2,6-di(isopropyl)phenyl]-1,1-diethyl-guanidinato}-(diethylamido)-dichloridozirconium(VI) **(Aa;** mixtures of isomers)

Method **A:**

Bis(diethylamido)-dichloridozirconium(IV)-bis(tetrahydrofurane) (0.036 g, 80 □mol) and Bis(2,6-diisopropylphenyl) carbodiimide (0.029 g, 80 □mol) were subsequently added to a Schlenk flask filled with 10 mL of toluene and stirred at RT. After 24 h the mixture was filtered and diluted with toluene to reach 40 mL. This solution was used in polymerisation without further purification.

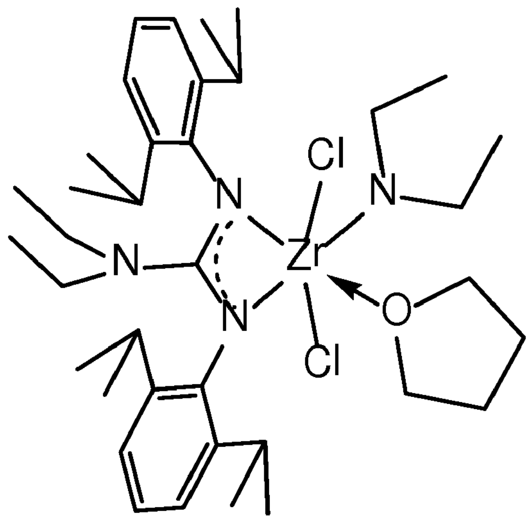

Alternative Method **B** can be used: Bis(diethylamido)-dichloridozirconium(IV)-bis(tetrahydrofurane) (0.036 g, 80 □mol) and (Z) -2,3-bis(2,6-diisopropylphenyl)-1,1-diethylguanidine (0.035 g, 80 □mol) were subsequently added to a Schlenk flask filled with 10 mL of toluene and stirred at RT. After 24 h the mixture was filtered and diluted with toluene to reach 40 mL. This solution was used in polymerisation without further purification.

*Comparative Example 2*

General description of ethylene polymerisation experiments for Runs 1 - 6

The catalytic ethylene polymerisation reactions were performed in a 250 mL glass autoclave (Buechi) in semi-batch mode (ethylene was added by replenishing flow to keep the pressure constant). The reactor was ethylene flow controlled and equipped with separated toluene, catalyst and co-catalyst injection systems. During a polymerisation run the pressure and the reactor temperature were kept constant while the ethylene flow was monitored continuously. In a typical semi-batch experiment, the autoclave was evacuated and heated for 1 h at 80 °C prior to use. The reactor was then brought to desired temperature, stirred at 1000 rpm and charged with 150 mL of toluene. After pressurizing with ethylene to reach 2 bar total pressure the autoclave was equilibrated for 10 min. Successive TEAL co-catalyst solution, activator (perfluorophenylborate) and 1 mL of a zirconium pre-catalyst stock solution in toluene was injected, to start the reaction.

After the desired reaction time the reactor was vented and the residual aluminium alkyls were destroyed by addition of 50 mL of ethanol. Polymeric product was collected, stirred for 30 min in acidified ethanol and rinsed with

ethanol and acetone on a glass frit. The polymer was initially dried on air and subsequently in vacuum at 80°C. Oligomeric product was collected by washing the toluene solution with water and removing the solvent under reduced pressure. The oily product was analyzed by GC-MS.

**Table 1.** Ethylene polymerisation with Zr pre-catalyst **Aa,** TEAL co-catalyst and perfluorophenylborate activator.[a]

| Entry | Precat | Al/Zr | t [min] | $m_{product.}$ [g] | Activity [$kg_{PE}mol_{cat}^{-1}h^{-1}bar^{-1}$] | $M_n$ [$kgmol^{-1}$] | $M_w/M_n$ |
|---|---|---|---|---|---|---|---|
| **1** | **Aa** | 250 | 15 | 1.68 | 1680 | 1300 | 1.9 |
| **2** | **Aa** | 500 | 15 | 1.70 | 1700 | 1140 | 1.5 |
| **3** | **Aa** | 750 | 15 | 3.54 | 3540 | 1030 | 1.5 |
| **4** | **Aa** | 1000 | 15 | 6.48 | 6480 | 1290 | 1.3 |
| **5** | **Aa** | 10000 | 30 | 6.50 | 3270 | 350 | 1.3 |
| **6** | **Aa** | 10000 | 60 | 21.00 | 5250 | 370 | 1.5 |

[a]Pre-catalyst: 2.0 μmol; ammonium borate: 2.2 μmol $[R_2N(CH_3)H]^+[B(C_6F_5)_4]^-$ ($R = C_{16}H_{33}$-$C_{18}H_{37}$), Zr/B = 1/1.1; toluene: 150 mL; T = 50°C, p = 2 bar.

*Example 1*: *Synthesis of 2,3-Bis(2,6-diisopropylphenyl)-1,1-diethylguanidinato diethylamino trichlorido zirconium(IV)* ***(Ia)***

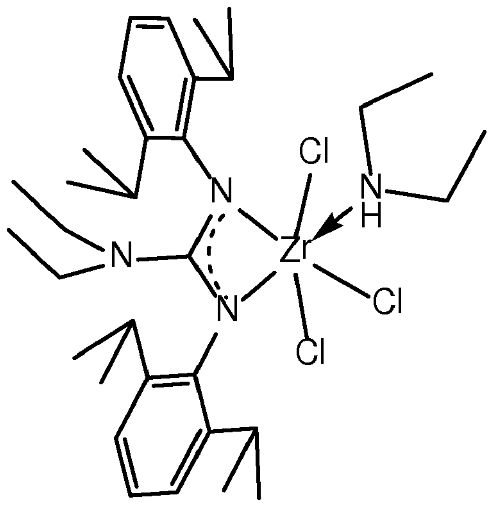

2,3-Bis(2,6-diisopropylphenyl)-1,1-diethylguanidine (2.55 g, 5.85 mmol) and diethylamido-trichloridozirconium(IV) etherate (2.01 g, 5.85 mmol) were dissolved in toluene (100 mL) and stirred overnight. Diethylamine (0.86 mg, 11.16 mmol) was added to the filtered reaction solution and the mixture was stirred for one hour. After filtration and concentration of the reaction solution, colourless crystals could be obtained at -30°C. $^1H$ NMR (300 MHz, $C_6D_6$): δ = 0.15 (t, 6H, $CH_3$), 0.67 (t, 6H, $CH_3$), 1.26 (d, 12H, $CH_3$), 1.59 (d, 12H, $CH_3$), 2.49 (s, br, 4H, $CH_2$), 2.77 (q, 4H, $CH_2$), 3.60 (s, 1 H, NH), 3.91 (m, 4H, CH), 7.13 (d, 6H, $CH_{arom}$) ppm.

*Example 2*: *Synthesis of 2,3-bis(2,6-diisopropylphenyl)-C-(cis-2,6-dimethylpiperidyl)guanidinato diethylamido trichlorido zirconium(IV)* ***(Id)***

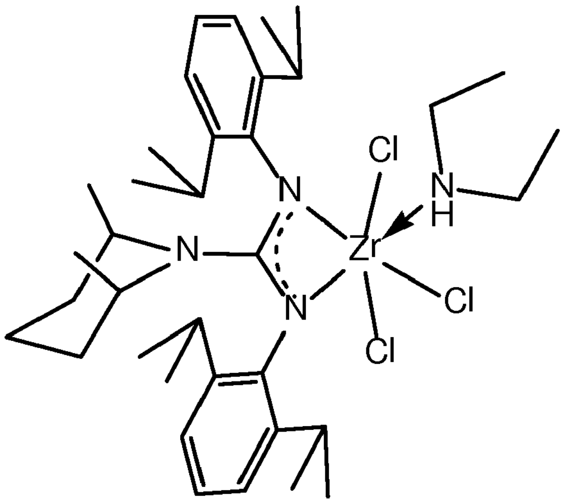

2,3-bis(2,6-diisopropylphenyl)-C-(*cis*-2,6-dimethylpiperidyl)guanidine (11.2 g, 23.5 mmol) and diethylamido-trichloridozirconium(IV) etherate (8.1 g, 23.5 mmol) were dissolved in toluene (300 mL) and stirred overnight. The reaction solution is filtered diethylamine (3.0 ml, 28.7 mmol) is added and stirred for one hour. After filtration and concentration of the reaction solution, colourless crystals could be obtained at -30°C. $^1H$ NMR (300 MHz, $C_6D_6$): $\delta$ = 0.73 (d, 6 H, $CH_3$); 0.75 (t, 6 H, $CH_3$); 1.29-1.72 (m, 6 H, $CH_2$); 1.04 (d, 6 H, $CH_3$); 1.40 (d, 6 H, $CH_3$); 1.47 (d, 6 H, $CH_3$); 1.71 (d, 6 H, $CH_3$); 2.54 (q, 4 H, $CH_2$); 3.23 (s, 1 H, NH); 3.99 (sept, 4 H, CH); 3.60-3.74 (m, 2 H, CH); 6.93-7.19 (m, 6 H, CHarom) ppm.

*Example 3*: *Synthesis of 2,3-Bis(2,6-diisopropylphenyl)-1,1-diethylguanidinato di-ethylamido-dimethanido zirconium(IV) (**IId**)*

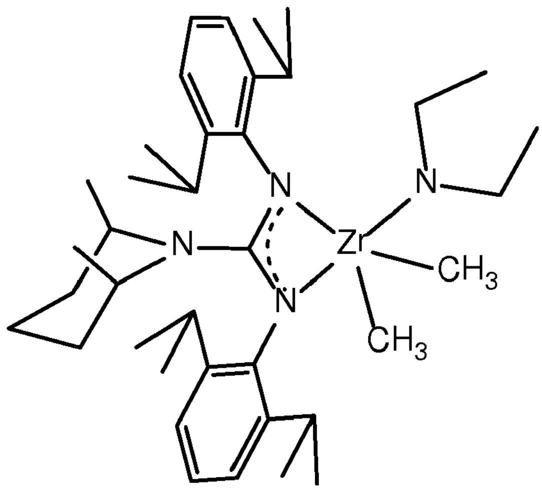

To a suspension of bis(2,6-diisopropylphenyl)-C-(*cis*-2,6-dimethyl-piperidyl)guanidinato-diethylamido-trichlorido-zirconium (2.5 g, 3.4 mmol) in ether (50 mL) methylmagnesium chloride (3 M in THF, 4.9 mL, 14.7 mmol) was added dropwise at -78°C. The mixture was warmed to room temperature and stirred overnight. Storage of the concentrated filtrate at -30 °C led to colourless crystals. Yield 1.9 g (85 %). $^1H$ NMR (300 MHz, $C_6D_6$): $\delta$ = 0.53 (s, 6 H, $CH_3$); 0.74 (d, 6 H, $CH_3$); 0.90 (t, 6 H, $CH_3$); 0.80-1.46 (m, 6 H, $CH_2$); 1.09 (d, 6 H, $CH_3$); 1.23 (d, 6 H, $CH_3$); 1.29 (d, 6 H, $CH_3$); 1.37 (d, 6 H, $CH_3$); 3.24 (q, 4 H, $CH_2$); 3.63 3.99 (sept, 4 H, CH); 3.88-3.98 (m, 2 H, CH); 7.04-7.14 (m, 6 H, CHarom) ppm.

*Example 4*

General description of ethylene polymerisation experiments for Runs 7 - 15

The catalytic ethylene polymerisation reactions were performed in a 250 mL glass autoclave (Buechi) in semi-batch mode (ethylene was added by replenishing flow to keep the pressure constant). The reactor was ethylene flow controlled and equipped with separated toluene, catalyst and co-catalyst injection systems. During a polymerisation run the pressure and the reactor temperature were kept constant while the ethylene flow was monitored continuously.

In a typical semi-batch experiment, the autoclave was evacuated and heated for 1 h at 80 °C prior to use. The reactor was then brought to desired temperature, stirred at 1000 rpm and charged with 150 mL of toluene. After pressurizing with ethylene to reach 2 bar total pressure the autoclave was equilibrated for 10 min. Successive TEAL co-catalyst solution, activator (perfluorophenylborate) and 1 mL of a zirconium pre-catalyst stock solution in toluene was injected, to start the reaction. After the desired reaction time the reactor was vented and the residual aluminium alkyls were destroyed by addition of 50 mL of ethanol. Polymeric product was collected, stirred for 30 min in acidified ethanol and rinsed with ethanol and acetone on a glass frit. The polymer was initially dried on air and subsequently in vacuum at 80°C. Oligomeric product was collected by washing the toluene solution with water and removing the solvent under reduced pressure. The oily product was analyzed by GC-MS.

**Table 2.** Ethylene polymerisation with Zr pre-catalysts example **1-3,** TEAL co-catalyst and perfluorophenylborate activator.[a]

| Entry | Precat | Al/Zr | t [min] | $m_{roduct.}$ [g] | Activity [$kg_{PE}mol_{cat}^{-1}h^{-1}bar^{-1}$] | $M_n$ [$kgmol^{-1}$] | $M_w/M_n$ |
|---|---|---|---|---|---|---|---|
| 7 | **Ia** | 500 | 15 | 0.63 | 1150 | 650 | 1.14 |
| 8[b] | **Ia** | 5000 | 15 | 1.13 | 1080 | liquid[d] | - |
| 9 | **Id** | 500 | 15 | 1.45 | 2900 | 1181 | 1.5 |
| 10 | **Id** | 1000 | 15 | 4.58 | 9160 | 860 | 1.5 |
| 11[c] | **Id** | 1000 | 15 | 10.87 | 21800 | 2590 | 2.5 |
| 12[c,f] | **Id** | 72000 | 60 | 68.2 | 14200 | 650 | 1.6 |
| 13[b,f] | **Id** | 79000 | 15 | 28.1 | 14080 | liquid[d] | - |
| 14[b,e] | **Id** | 75000 | 15 | 28.5 | 17070 | 280 | 1,6 |
| 15 | **IId** | 500 | 15 | 3.7 | 7400 | 12450 | 1.9 |

[a]Precatalyst: 1.0 μmol; ammonium borate: 1.1 μmol $[R_2N(CH_3)H]^+[B(C_6F_5)_4]^-$ (R = $C_{16}H_{33}$-$C_{18}H_{37}$), Zr/B = 1/1.1; toluene: 150 mL; T = 50°C, p = 2 bar; t = 15 min.
[b]Precatalyst: 2.0 μmol. [c]anilinium borate: 1.1 mmol $[PhN(CH_3)_2H]^+[B(C_6F_5)_a]^-$. [d]oligomeric products. [e]3 bar ethylene. [f]4 bar ethylene.

*Example 5*: *Synthesis of Di-μ-chlorido-bis[2,3-bis(2,6-diisopropylphenyl)-1,1-diethylguanidinato]-tetrachlorido-dizirconium(IV) (**III**)*

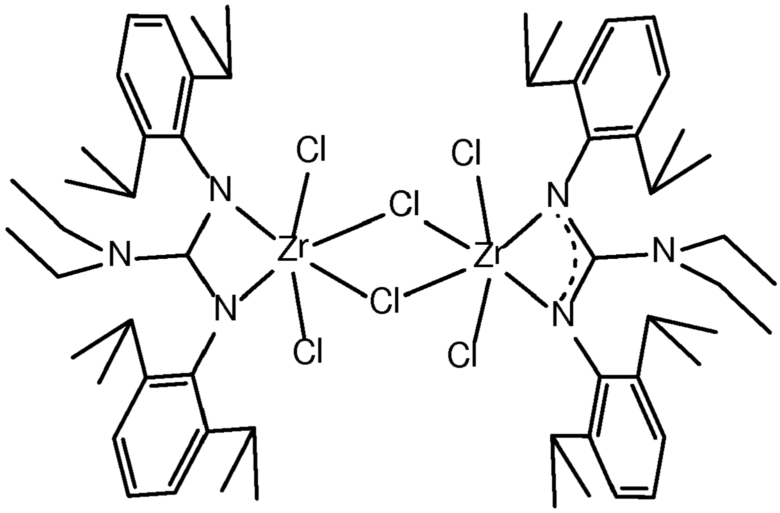

**III**

Diethylamido-trichloridozirconium(IV) etherate (0.68 g, 2.0 mmol) and Bis(2,6-diisopropylphenyl) carbodiimide (0.55 g, 1.5 mmol were dissolved in toluene (100 mL) and stirred overnight at 60°C. After filtration and concentration of the reaction solution, colourless crystals were obtained at -30°C. $^1H$ NMR (300 MHz, $C_6D_6$): $\delta$ = 0.20 (t, 6H, $CH_3$), 1.18 (d, 12H, $CH_3$), 1.50 (d, 12H, $CH_3$), 2.59 (q, 4H, $CH_2$), 3.55 (m, 4H, CH), 7.06 (d, 6H, $CH_{arom}$) ppm. $^{13}C$ NMR (75.4 MHz, $C_6D_6$): $\delta$ = 20.5 ($CH_3$), 25.3 ($CH_3$), 37.8 (CH), 48.7 ($CH_2$), 121.1 (Carom), 123.6 (Carom), 125.6 (Carom) ppm.

*Example 6 Synthesis of 2,3-Bis(2,6-diisopropylphenyl)-1,1-diethylguanidinato trimethanido zirconium(IV)* ***(IV)***

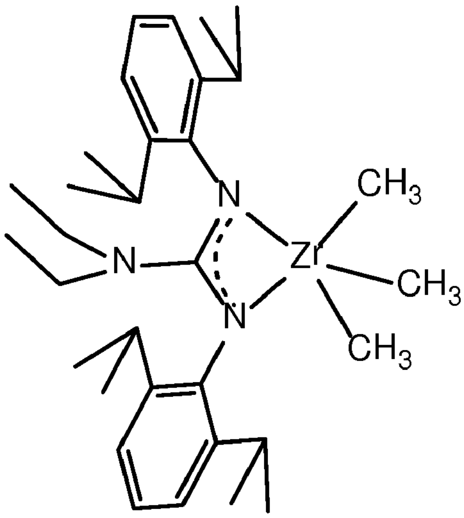

To a suspension of dimeric $\mu^2$-Chlorido-[2,3-bis(2,6-diisopropylphenyl)-1,1-diethylguanidinato]trichloridozirconium(IV) (1176 mg, 0.93 mmol) in hexane (50 mL) methylmagnesium chloride (1.9 mL, 5.58 mmol) was added dropwise at-78°C. The mixture was warmed to room temperature and stirred overnight. Storage of the concentrated filtrate at -30 °C led to colourless crystals. Yield 903 mg (85 %). $^1H$ NMR (300 MHz, $C_6D_6$): $\delta$ = 0.26 [t, J = 7.1 Hz, 6 H, $N(CH_2CH_3)_2$]; 0.86 [s, 9 H, $Zr(CH_3)_3$]; 1.24 [d, J = 6.9Hz, 12 H, $CH(CH_3)_2$]; 1.36 [d, J = 7.1 Hz, 12 H, $CH(CH_3)_2$]; 2.74 [q, J = 7.1 Hz, 4 H, $N(CH_2CH_3)_2$]; 3.62 [sept, J = 6.8 Hz, 4 H, $CH(CH_3)_2$]; 7.09 (s, 6 H, ArH) ppm. $^{13}C$ NMR (75.4 MHz, $C_6D_6$): $\delta$ = 11.3 [$N(CH_2CH_3)_2$]; 24.0 [$CH(CH_3)_2$]; 25.9 [$CH(CH_3)_2$]; 28.5 [$CH(CH_3)_2$]; 40.8 [$N(CH_2CH_3)_2$]; 51.4 [$Zr(CH_3)_3$]; 124.3, 125.5, 142.7, 143.3 (ArC); 169.5 (NCN) ppm.

*Example* 7: Synthesis of Di-μ-methylene-bis[2,3-bis(2,6-diisopropylphenyl)-1,1-diethylguanidinato]-dimethanido-dizirconium(IV) (**V**)

To a suspension of Di-μ-chlorido-bis[2,3-bis(2,6-diisopropylphenyl)-1,1-diethylguanidinato]-tetrachlorido-dizirconium(IV) (1.92 g, 1.52 mmol) in THF (30 mL) methylmagnesium chloride (3.05 mL, 9.12 mmol) was added dropwise at-78°C. The mixture was warmed to room temperature and stirred overnight. Solvent was removed under reduced pressure and the residue extracted twice with hexane (2x20 mL). Storage of the concentrated filtrate at -30 °C led to light yellow crystals. Yield 1.68 g (88 %). $^1H$ NMR (300 MHz, C6D6): δ = 0.21-0.27 (m, 6H, $N(CH_2CH_3)_2$); 0.58 (s, 3H, $Zr(CH_3)_3$); 1.26 (d, 6H, J=6.8Hz, $CH(CH_3)_2$); 1.31 (d, 6H, J=6.8Hz, $CH(CH_3)_2$); 1.39 (d, 6H, J=6.7Hz, $CH(CH_3)_2$); 1.48 (d, 6H, J=6.7Hz, $CH(CH_3)_2$); 2.76 (m, 4H, $N(CH_2CH_3)_2$); 3.62 (sept., 2H, J=6.8Hz, $CH(CH_3)_2$); 3.83 (sept., 2H, J=6.8Hz, $CH(CH_3)_2$); 5.25 (s, 2H, $Zr(CH_2)Zr$); 7.05 (m, 6H, Ar*H*) ppm.

*Example 8*

General description of ethylene polymerisation experiments for Runs 16 - 21

The catalytic ethylene polymerisation reactions were performed in a 250 mL glass autoclave (Buechi) in semi-batch mode (ethylene was added by replenishing flow to keep the pressure constant). The reactor was ethylene flow controlled and equipped with separated toluene, catalyst and co-catalyst injection systems. During a polymerisation run the pressure and the reactor temperature were kept constant while the ethylene flow was monitored continuously. In a typical semi-batch experiment, the autoclave was evacuated and heated for 1 h at 80 °C prior to use. The reactor was then brought to desired temperature, stirred at 1000 rpm and charged with 150 mL of toluene. After pressurizing with ethylene to reach 2 bar total pressure the autoclave was equilibrated for 10 min. Successive TEAL co-catalyst solution, activator (perfluorophenylborate) and 1 mL of a 0.001 M zirconium pre-catalyst stock solution in toluene was injected, to start the reaction. After the desired reaction time the reactor was vented and the residual aluminium alkyls were destroyed by addition of 50 mL of ethanol. Polymeric product was collected, stirred for 30 min in acidified ethanol and rinsed with ethanol and acetone on a glass frit. The polymer was initially dried on air and subsequently in vacuum at 80°C. Oligomeric product was collected by washing the toluene solution with water and removing the solvent under reduced pressure. The oily product was analyzed by GC-MS.

**Table 3.** Ethylene polymerisation with Zr pre-catalyst **III** and **IV,** TEAL co-catalyst and perfluorophenylborate activator.[a]

| Entry | Precat | Al/Zr | t [min] | $m_{product.}$ [g] | Activity [$kg_{PE}mol_{cat}^{-1}h^{-1}bar^{-1}$] | $M_n$ [$kgmol^{-1}$] | $M_w/M_n$ |
|---|---|---|---|---|---|---|---|
| 16[b] | **III** | 2000 | 15 | 1.00 | 500 | 560 | 1.2 |
| 17[b] | **III** | 1000 | 15 | 1.40 | 700 | 780 | 1.5 |
| 18[b] | **III** | 500 | 15 | 3.24 | 1620 | 990 | 1.5 |
| 19 | **IV** | 2000 | 15 | 12.73 | 25480 | 3000 | 1.5 |
| 20[c,e] | **IV** | 72000 | 22 | 28.13 | 25598 | liquid[d] | - |
| 21 | **IV** | 1000 | 15 | 13.38 | 26800 | 2480 | 1.9 |

[a]Precatalyst: 1.0 μmol; ammonium borate: 1.1 μmol $[R_2N(CH_3)H]^+[B(C_6F_5)_4]^-$ (R = $C_{16}H_{33}$-$C_{18}H_{37}$), Zr/B = 1/1.1; toluene: 150 mL; T = 50°C, p = 2 bar; t = 15 min.
[b] Precatalyst: 2.0 μmol, t = 30 min. [c]anilinium borate: 1.1 mmol $[PhN(CH_3)_2H]^+[B(C_6F_5)_4]^-$. [d]oligomeric products. [e]3 bar ethylene.

*Example 9*

General description of ethylene polymerisation experiments for Entries 22 - 24

The catalytic ethylene polymerisation reactions were performed in a 250 mL glass autoclave (Buechi) in semi-batch mode (ethylene was added by replenishing flow to keep the pressure constant). The reactor was ethylene flow controlled and equipped with separated toluene, catalyst and co-catalyst injection systems. During a polymerisation run the pressure and the reactor temperature were kept constant while the ethylene flow was monitored continuously. In a typical semi-batch experiment, the autoclave was evacuated and heated for 1 h at 80 °C prior to use. The reactor was then brought to desired temperature, stirred at 1000 rpm and charged with 150 mL of toluene. After pressurizing with ethylene to reach 2 bar total pressure the autoclave was equilibrated for 10 min. Successive TEAL co-catalyst solution, activator (perfluorophenylborate), Diethyl aluminium chloride and 1 mL of a 0.001 M zirconium pre-catalyst stock solution

in toluene was injected, to start the reaction. After the desired reaction time the reactor was vented and the residual aluminium alkyls were destroyed by addition of 50 mL of ethanol. Polymeric product was collected, stirred for 30 min in acidified ethanol and rinsed with ethanol and acetone on a glass frit. The polymer was initially dried on air and subsequently in vacuum at 80°C.

**Table 4.** Ethylene polymerisation with Zr pre-catalyst **IV** in presence of DEAC, TEAL co-catalyst and perfluorophenylborate activator.[a]

| Entry | TEAL Al/Zr | DEAC Cl/Zr | $M_{product}$ [g] | Activity [$kg_{PE}mol_{cat}^{-1}h^{-1}bar^{-1}$] | $M_n$ [$kgmol^{-1}$] | $M_w/M_n$ |
|---|---|---|---|---|---|---|
| 22 | 2000 | 1 | 8.64 | 17300 | 1790 | 1.5 |
| 23 | 2000 | 3 | 3.11 | 6230 | 950 | 1.6 |
| 24 | 2000 | 6 | 0.88 | 1750 | 630 | 1.5 |

[a]Precatalyst **IV**: 1.0 μmol; ammonium borate: 2.2 μmol $[R_2N(CH_3)H]^+[B(C_6F_5)_4]^-$ (R = $C_{16}H_{33}$ - $C_{18}H_{37}$), Zr/B = 1/1.1; toluene: 150 mL; T = 50°C, p = 2 bar; t = 15 min.

Example S1 (in-situ)

*General description of ethylene polymerisation experiments for Entries 25 + 26 (Table 5)*

The catalytic ethylene polymerisation reactions were performed in a 250 mL glass autoclave (Buechi) in semi-batch mode (ethylene was added by replenishing flow to keep the pressure constant). The reactor was ethylene flow controlled and equipped with separated toluene, catalyst and co-catalyst injection systems. During a polymerisation run the pressure and the reactor temperature were kept constant while the ethylene flow was monitored continuously.

In a typical semi-batch experiment, the autoclave was evacuated and heated for ½ h at 80 °C prior to use. The reactor was then brought to desired temperature, stirred at 500 rpm and charged with 150 mL of toluene. After pressurizing with ethylene to reach 2 bar total pressure the autoclave was equilibrated for 10 min. Successive chain transfer agent, activator, and 1 mL of a 0.001 M pre-catalyst stock solution in toluene was injected, to start the reaction. After 15 min reaction time the reactor was vented and the residual CSA alkyls were destroyed by addition of 20 mL of ethanol. Polymeric product was collected by filtration at 50°C, washed with acidified ethanol and rinsed with ethanol and acetone on a glass frit. The polymer was initially dried on air and subsequently in vacuum at 50°C. The soluble residue was analyzed by GC and /or GC-MS.

Example S2 (in-situ): General description of ethylene polymerisation experiments for Entries 27 + 28 (Table 5)

The catalytic ethylene polymerisation reactions were performed in a 250 mL glass autoclave (Buechi) in semi-batch mode (ethylene was added by replenishing flow to keep the pressure constant). The reactor was ethylene flow controlled and equipped with separated toluene, catalyst and co-catalyst injection systems. During a polymerisation run the pressure and the reactor temperature were kept constant while the ethylene flow was monitored continuously. In a typical semi-batch experiment, the autoclave was evacuated and heated for ½ h at 80 °C prior to use. The reactor was then brought to desired temperature, stirred at 500 rpm and charged with 150 mL of toluene. After pressurizing with ethylene to reach 2 bar total pressure the autoclave was equilibrated for 10 min. Successive chain transfer agent, activator and chain displacement catalyst, all dissolved in toluene, were injected, to start the reaction. After 15 min reaction time the reactor was vented and the residual CSA alkyls were destroyed by addition of 20 mL of ethanol. The toluene solution was analyzed by GC and /or GC-MS.

Example S3 (in-situ): General description of ethylene polymerisation experiments for Runs 29 - 38 (Table 6)

The catalytic ethylene polymerisation reactions were performed in a 250 mL glass autoclave (Buechi) in semi-batch mode (ethylene was added by replenishing flow to keep the pressure constant). The reactor was ethylene flow controlled and equipped with separated toluene, catalyst and co-catalyst injection systems. During a polymerisation run the pressure and the reactor temperature were kept constant while the ethylene flow was monitored continuously. In a typical semi-batch experiment, the autoclave was evacuated and heated for ½ h at 80 °C prior to use. The reactor was then brought to desired temperature, stirred at 500 rpm and charged with the desired amount of toluene. After pressurizing with ethylene to reach the desired total pressure the autoclave was equilibrated for 10 min. Successive chain transfer agent, activator, chain displacement catalyst and pre-catalyst, all dissolved in toluene, were injected, to start the reaction. After the appropriate reaction time the reactor was vented and the residual CSA alkyls were destroyed by addition of

20 mL of ethanol. Polymeric product was collected by filtration at 50°C, washed with acidified ethanol and rinsed with ethanol and acetone on a glass frit. The polymer was initially dried on air and subsequently in vacuum at 50°C. The soluble residue was analyzed by GC and /or GC-MS.

Example S4 (in-situ)

The catalytic ethylene polymerisation reaction was performed in a 1000 mL glass autoclave (Buechi) in semi-batch mode (ethylene was added by replenishing flow to keep the pressure constant). The reactor was ethylene flow controlled and equipped with separated toluene, catalyst and co-catalyst injection systems. During the polymerisation run the pressure and the reactor temperature were kept constant while the ethylene flow was monitored continuously. In a typical semi-batch experiment, the autoclave was evacuated and heated for ½ h at 100 °C prior to use. The reactor was then brought to desired temperature, stirred at 500 rpm and charged with 300 mL toluene. After pressurizing with ethylene to reach the desired total pressure the autoclave was equilibrated for 10 min. 1000 μmol TEAL, 4 μmol of 2,3-Bis(2,6-diisopropylphenyl)-1,1-diethylguanidinato trimethanido zirconium(IV), 6 μmol of Dimethylaniliniumborate and 8 μmol of Bis(cyclooctadienyl)nickel(0), was added to start the reaction. 30 g of ethylene was dosed into the reactor.

The temperature was maintained at 60 °C. After the appropriate reaction time the reactor was vented and the residual TEAL was destroyed by addition of 20 mL of ethanol. A sample is taken from the solution and analyzed via GC with nonane as internal standard.

**Table 5.** Comparative ethylene oligomerisation with $GuaTiMe_3$ (**I**) and $GuaZrMe_3$ (**IV**) precatalysts or $Ni(stea)_2$ and $Ni(COD)_2$ CDC's only.[a]

| Entry | precat. [μmol] | CSA [μmol] | CDC [μmol] | C2 consump. [l] | yield [g] | Activity $\left[\frac{Kg_{PE}}{mol_{cat} \cdot h \cdot bar}\right]$ | m(C4) [g] | m(C6) [g] | m(C8) [g] | m(C10) [g] | m(C12) [g] | $m_{solid}$ [g] | $M_n$ $\left[\frac{g}{mol}\right]$ | $M_w/M_n$ |
|---|---|---|---|---|---|---|---|---|---|---|---|---|---|---|
| 25 | I (Ti)* 2 | 10000 | - | 1.92 | 3.36 | 3360 | - | - | - | - | - | 1.92 | 1120 | 1.6 |
| 26 | IV (Zr) 1 | 1000 | - | 12.14 | 15.18 | 30360 | - | - | - | - | - | 15.18 | 2480 | 1.9 |
| 27 | - | 1000 | $Ni(stea)_2$ 2 | 0.00 | 0 | 0 | - | - | - | - | - | - | - | - |
| 28 | - | 1000 | $Ni(COD)_2$ 2 | 0.10 | 0.12 | 125 | 0.10 | - | - | - | - | - | - | - |

[a]activator: $[Me_2NPhH][B(C_6F_5)_4]$, Zr/B = 1/2; toluene: 150 mL; $p_{ethylene}$ = 2 bar; t = 15 min;*2,3-Bis(2,6-diisopropylphenyl)-1,1-diethylguanidinato trimetha-nido titanium(IV) (GuaTiMe3, I, J. Obenauf, W. P. Kretschmer, R. Kempe, Eur. J.Inorg. Chem. 2014, 1446-1453) was prepared according to published procedures.

**Table 6.** Ethylene oligomerisation with $GuaTiMe_3$ (**I**) and $GuaZrMe_3$ (IV) precatalysts in presence of CDC's.[a]

| Entry | precat. [µmol] | CSA [µmol] | CDC [µmol] | C2consump. [l] | yield [g] | Activity $\left[\frac{Kg_{PE}}{mol_{cat} \cdot h \cdot bar}\right]$ | m(C6) [g] | m(C8) [g] | m(C10) [g] | m(C12) [g] | m(C14) [g] | $m_{solid}$ [g] | $M_n$ $\left[\frac{g}{mol}\right]$ | $M_w/M_n$ |
|---|---|---|---|---|---|---|---|---|---|---|---|---|---|---|
| 29 | I (Ti)* 2 | 10000 | $Ni(COD)_2$ 2 | 6.68 | 8.35 | 8350 | - | - | - | - | - | 6.33 | 840 | 1.5 |
| 30 | IV (Zr) 1 | 1000 | $Ni(acac)_2$ 7.8 | 10.40 | 13.00 | 26000 | 1.64 | 1.42 | 1.20 | 1.01 | 0.83 | 0.18 | | |
| 31 | IV (Zr) 1 | 1000 | $Ni(stea)_2$ 1 | 10.77 | 13.46 | 26925 | 0.42 | 0.43 | 0.43 | 0.44 | 0.45 | 6.90 | 810 | 1.6 |
| 32[b] | IV (Zr) 1 | 1000 | $Ni(stea)_2$ 2 | 17.50 | 21.88 | 21875 | 1.67 | 1.58 | 1.50 | 1.38 | 1.19 | 1.69 | 740 | 1.5 |
| 33[b] | IV (Zr) 1 | 1000 | $Ni(COD)_2$ 1 | 17.23 | 21.54 | 21540 | 1.37 | 1.32 | 1.24 | 1.08 | 0.97 | 4.88 | 790 | 1.5 |
| 34[b] | IV (Zr) 1 | 520 | $Ni(COD)_2$ 2 | 15.20 | 19.00 | 19000 | 0.72 | 0.72 | 0.72 | 0.71 | 0.70 | 0.22 | 370 | 41.5[g] |
| 35[b] | IV (Zr) 1 | 330 | $Ni(COD)_2$ 2 | 15.10 | 18.88 | 18875 | 1.01 | 0.99 | 0.98 | 0.92 | 0.86 | 0.40 | 520 | 133.7 [g] |
| 36[b] | IV (Zr) 1 | 300 | $Ni(COD)_2$ 2 | 15.00 | 18.75 | 18750 | 1.04 | 1.02 | 0.98 | 0.94 | 0.87 | 1.95 | 390 | 2.3 |
| 37[c] | IV (Zr) 1 | 280 | $Ni(COD)_2$ 2 | 16.80 | 21.00 | 21000 | 0.91 | 0.91 | 0.92 | 0.91 | 0.89 | 8.34 | 390 | 2.0 |
| 38[d] | IV (Zr) 1 | 650 | $Ni(COD)_2$ 2 | 0.80 | 1.50 | 3000 | 0.36 | 0.26[e] | - | 0.16 | 0.10[f] | 0.05 | n.d. | n.d. |

[a]activator: $[Me_2NPhH][B(C_6F_5)_4]$, Zr/B = 1/2; toluene: 150 mL; $p_{ethylene}$ = 2 bar; t = 15 min. [b]t = 30 min, CSA = . Yield was calculated from ethylene consumption, m(C4) was not determined. [c]$p_{ethylene}$ = 4 bar. [d]$p_{propylene}$ = 2 bar. [e]$C_9$. [f]$C_{15}$. [g]bimodal;* 2,3-Bis(2,6-diisopropylphenyl)-1,1-diethylguanidinato trimethanido titanium(IV) (GuaTiMe3, I, J. Obenauf, W. P. Kretschmer, R. Kempe, Eur. J.Inorg. Chem. 2014, 1446-1453) was prepared according to published procedures.

Waxy product was collected by filtration (0.2 μm) at 50°C, washed with acidified ethanol and rinsed with ethanol and acetone on a glass frit. The filtrate was initially dried on air and subsequently in vacuum at 50°C and analyzed via GPC. The permeate was analyzed by GC and /or GC-MS.

The effect of applied pressure and temperature are shown in Fig. 7 and 8.

Example S5 (sequential process)

The catalytic ethylene polymerisation reaction was performed in a 1000 mL glass autoclave (Buechi) in semi-batch mode (ethylene was added by replenishing flow to keep the pressure constant). The reactor was ethylene flow controlled and equipped with separated toluene, catalyst and co-catalyst injection systems. During a polymerisation run the pressure and the reactor temperature were kept constant while the ethylene flow was monitored continuously. In a typical semi-batch experiment, the autoclave was evacuated and heated for ½ h at 100 °C prior to use. The reactor was then brought to desired temperature, stirred at 500 rpm and charged with 300 mL toluene. After pressurizing with ethylene to reach the desired total pressure the autoclave was equilibrated for 10 min. 40000 μmol TEAL, 4 μmol of 2,3-Bis(2,6-diisopropylphenyl)-1,1-diethylguanidinato trimethanido zirconium(IV), 4 μmol of trioctylammonium borate was added to start the reaction. 22 g of ethylene was dosed into the reactor and the temperature was maintained at 60 °C.

After the desired amount of ethylene was consumed the reactor was depressurized and the reactor flushed with argon. Subsequently the temperature was raised to 100 °C for 1 hour. 8 μmol Bis(cyclooctadienyl)nickel(0), was added to the reactor via a syringe. A temperature of 120 °C was set and maintained via a thermostat. The reactor was pressurized with ethylene again and the reaction monitored until no more ethylene was consumed. The residual TEAL was destroyed by addition of 20 mL of ethanol. A sample was taken from the solution and analyzed via GC with nonane as internal standard. Waxy product was collected by filtration (0.2 μm) at 50°C, washed with acidified ethanol and rinsed with ethanol and acetone on a glass frit. The filtrate was initially dried on air and subsequently in vacuum at 50°C and analyzed via GPC. The permeate was analyzed by GC and /or GC-MS.The distribution of the obtained linear α-olefins are shown in Fig. 9.

**Claims**

**1.** Process for the manufacture of oligomerised olefins by bringing in contact with each other

(a) one or more C2 to C8 olefins,
(b) a coordinative chain transfer polymerisation catalyst (CCTP catalyst) comprising one or more organo metallic transition metal compounds and one or more ligands,
(c) a chain shuttling agent (CSA) being one or more metal alkyls selected from the group II, XII and XIII,
(d) a chain displacement catalyst (CDC) being one or more member selected from the group consisting of a nickel salt, a cobalt salt, an organo metallic nickel complex and an organo metallic cobalt complex,

to form a growth composition thereby obtaining oligomerised olefins having an oligomerisation degree of 2 to 100, wherein the process also includes that (d) is brought in contact only at a later point in time with the growth composition when the oligomerisation has commenced or has come to an end and (b) is at least partially or completely transformed into an inactive reaction product or inactive degradation product.

**2.** The process according to claim 1, wherein the growth composition further comprises an activator for the coordinative chain transfer polymerisation catalyst (CCTP catalyst) being an aluminium or boron containing compound comprising at least one hydrocarbyl group.

**3.** The process according to claim 1 or claim 2 , wherein the olefin is one or more member selected from the group consisting of ethylene, propylene, 1-butene, 1-pentene and 1-hexene, preferably one or more member selected from the group ethylene, propylene or ethylene and propylene.

**4.** The process according to one or more of the preceding claims, wherein the one or more organo metallic transition metal compounds comprises one or two transition metals, preferably one transition metal, selected from

- group III,
- group IV, preferably Ti or Zr, most preferably Zr
- group V,
- group VI,

- group IX
- group X

of the periodic table.

**5.** The process according to one or more of the preceding claims, wherein one or two ligands are selected from cyclopentadienyl, indenyl, fluorine, diamide ligands, phenoxy-imine-ligand, indolide-imine-ligands, amidinate, guanidinate, amidopyridine, pyrrdinimine and alcoholate each optionally substituted.

**6.** The process according to one or more of the preceding claims, wherein the CCTP catalyst is deactivated during or after the oligomerisation by heating the growth composition, most preferably above 120°C or by bringing the CCTP catalyst in contact with a catalyst poison, preferably being a halogen containing compound, preferably an halogened hydrocarbyl aluminium.

**7.** The process according to one or more of the preceding claims wherein the chain shuttling agent (CSA) is a C1 to C30 hydrocarbyl metal compound, methylalumoxane or both, the metal being aluminium, zinc, magnesium, indium or gallium, preferably trihydrocarbyl aluminium, dihydrocarbyl magnesium or dihydrocarbyl zinc.

**8.** The process according to one or more of the preceding claims wherein the chain displacement catalyst (CDC) is selected from nickel halogenides, cobalt halogenides, nickel cyclooctadiene, cobalt cyclooctadiene, nickel acetylactonate, C1 to C30 carboxylic acid salts of nickel and mixtures thereof.

**9.** The process according to one or more of claims 2 to 8 wherein the activator is methyl aluminoxan, or a perfluorated aluminate or a boron containing compound or combinations thereof and the boron containing compound preferably comprises one or more members selected from the group consisting of tris(pentafluoro phenyl) borane, tetrakis(pentafluoro phenyl) borate, tris(tetrafluoro phenyl) borane and tetrakis(tetrafluoro phenyl) borate.

**10.** The process according to one or more of the preceding claims wherein the molar ratio of the coordinative chain transfer polymerisation catalyst (CCTP catalyst) to the chain shuttling agent (CSA) is 1 : > 50000 or 1 : 50 to 1 : 10000, except for methyl alumoxane as the CSA.

**11.** The process according to one or more of the preceding claims wherein the molar ratio of the coordinative chain transfer polymerisation catalyst (CCTP catalyst) to the chain displacement catalyst (CDC)

- prior or during the oligomerisation is 1 : 0.5 to 1 : 50, preferably 1 : 1 to 1 : 2, and
- after the oligomerisation the concentration of the chain displacement catalyst (CDC) is between 1 to 10000 ppm, preferably 1 to 100 ppm (w/w) relative to the growth composition.

**12.** The process according to one or more of claims 2 to 11 wherein the molar ratio of the coordinative chain transfer polymerisation catalyst (CCTP catalyst) to the activator is 1 : 1 to 1 : 4, preferably 1: 1,05 to 1: 2, except for methyl alumoxane.

**13.** The process according to one or more of the preceding claims wherein the growth composition further comprises a liquid reaction medium, the liquid reaction medium comprising aromatic hydrocarbons, particularly toluene, linear and/or branched C4 to C20 hydrocarbons and mixtures thereof; cyclic and alicyclic hydrocarbons such as cyclohexane, cycloheptane, and/or methylcyclohexane,.

**14.** The process according to one or more the preceding claims wherein the reaction is carried out at a C2 to C3 pressure of 0,2 to 60 bar, preferably 1 to 20 bar; most preferably 1 to 10 bar or a C4 pressure of 0,2 to 20 bar, preferably 1 to 10 bar.

**15.** The process according to one or more of the preceding claims, wherein the coordinative chain transfer polymerisation catalyst comprises as transition metal Ti, Zr or Hf and one ligand per metal of the following formula

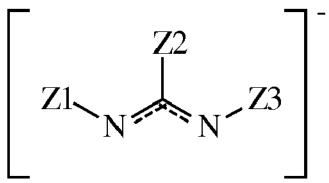

I,

the ligand being bound to the metal, wherein

Z1, Z2 and Z3 = are independently hydrocarbon or heteroatom containing hydrocarbon moieties, wherein the heteroatom, if present, for Z1 or Z3 is not directly adjacent to the N-atom and, wherein Z1, Z2 and Z3 independently from each other are optionally linked with one or more of each other.

**16.** The process according to one or more of the preceding claims, wherein the coordinative chain transfer polymerisation catalyst comprises as transition metal Ti, Zr or Hf and one ligand per metal having the following substructural element:

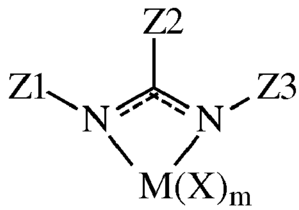

wherein

Z1 ,Z3 = each are independently from each other a di-ortho substituted aromatic moiety, each being independently hydrocarbon moieties or heteroatom containing hydrocarbon moieties, wherein the heteroatom, if present, is not directly adjacent to the N-atom,
Z2 = is a hydrocarbon moiety or a heteroatom containing hydrocarbon moiety, Z1, Z2 and Z3 independently from each other are optionally linked with one or more of each other, and
M = Titanium. Zirconium of Hafnium.

**17.** The process of claim 15 or 16 wherein Z2 is NR1 R2 with R1 and R2 independently from each other are C1 to C40 hydrocarbon moieties, optionally comprising one or more heteroatoms.

**18.** A process for the manufacture of a Di-μ-halogen-bridged bis guanidinato tetrahalogen di zirconium compound comprising bringing together in a solvent:

- a zirconium amido compound having the following formula

$$Zr\ (Hal)_3\ (NR^1R^2)\ \text{Etherate}$$

wherein

Hal is independent from each other Halogen, in particular Cl;
$R^1$,$R^2$ being C1 to C40 hydrocarbyl-, optionally comprising one or more heteroatoms, wherein the heteroatom is not adjacent to the N-Atom;
the etherate preferably being a di-(C1- to C6-)hydrocarbylether , in particular a di(C1- to C6-)alkylether, a di-(C2- or C3-)hydrocarbylether, in particular a Di(C2- or C3)alkylether;

and
an carbodiimid-compound having the following formula

$$(R^3)_xAr\text{-}N{=}C{=}N\text{-}Ar(R^4)_y$$

wherein

$R^3$,$R^4$= independent from each x, y is hydrocarbyl, in particular alkyl, or halogen, wherein R is preferably substituted at the 2 or 6 position of the aryl, and further wherein R is branched at the 2-position;
x,y = independent from each $R^3$or $R^4$0 to 3;
Ar = is Aryl, in particular Benzene.

**19.** The process according to claim 18 wherein the Di-μ-halogen-bridged bis guanidinato tetrahalogen di zirconium compound is

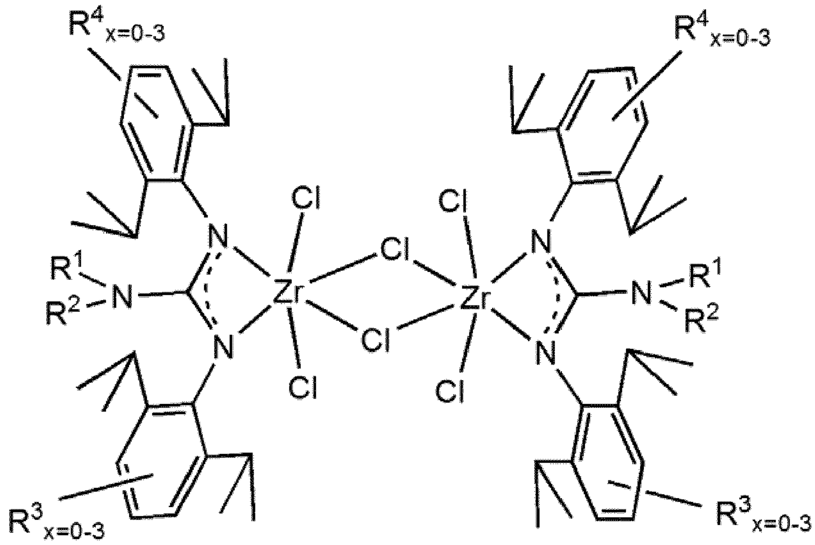

.

with

x = 0 to 3 independent from each $R^3$or $R^4$
$R^1$,$R^2$ ,$R^3$,$R^4$ as defined in claim 18.

**20.** A process for the manufacture of a zirconium guanidinato alkyl compound comprising the following steps:

- bringing together
a Di-μ-halogen-bridged bis guanidinato tetrahalogen di zirconium compound with a Grignard-Reagent, wherein the Grignard-Reagent is preferably used in a 2.8 to 3.2 times molar excess relative to the Zr.

**21.** The process of claim 20 wherein independent form each other the Di-μ-halogen-bridged bis guanidinato tetrahalogen di zirconium compound is obtainable by the process of any of claims 18 or 19;
the Grignard-Reagent is Alkyl Mg Hal, wherein
Hal is independent from each other Halogen, in particular Cl;
alkyl is C1 to C20 alkyl, in particular Methyl or Ethlyl.

**22.** The process of claim 20 or 21 wherein the zirconium guanidinato alkyl compound is

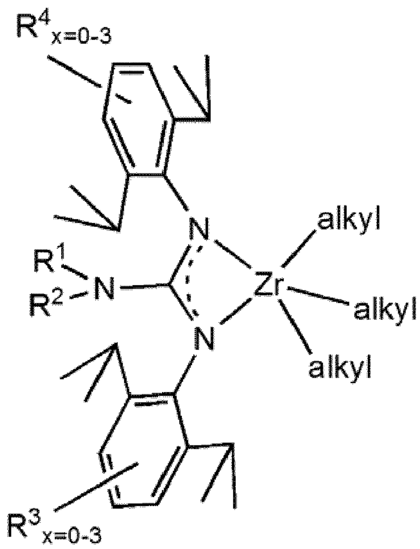

with

$R^1$,$R^2$ being C1 to C40 hydrocarbyl-, optionally comprising one or more heteroatoms, wherein the heteroatom is not adjacent to the N-Atom;
$R^3$,$R^4$= independent from each x, y is hydrocarbyl, in particular alkyl, or halogen, wherein R is preferably substituted at the 2 or 6 position of the aryl, and further wherein R is branched at the 2-position;
x = independent from each R $^3$or $R^4$ 0 to 3.

**24.** Use of the compound obtained according to the process of claims 20 to 23 in the process of any one of claims 1 to 17 as a CCTP catalyst.

**Fig. 1**

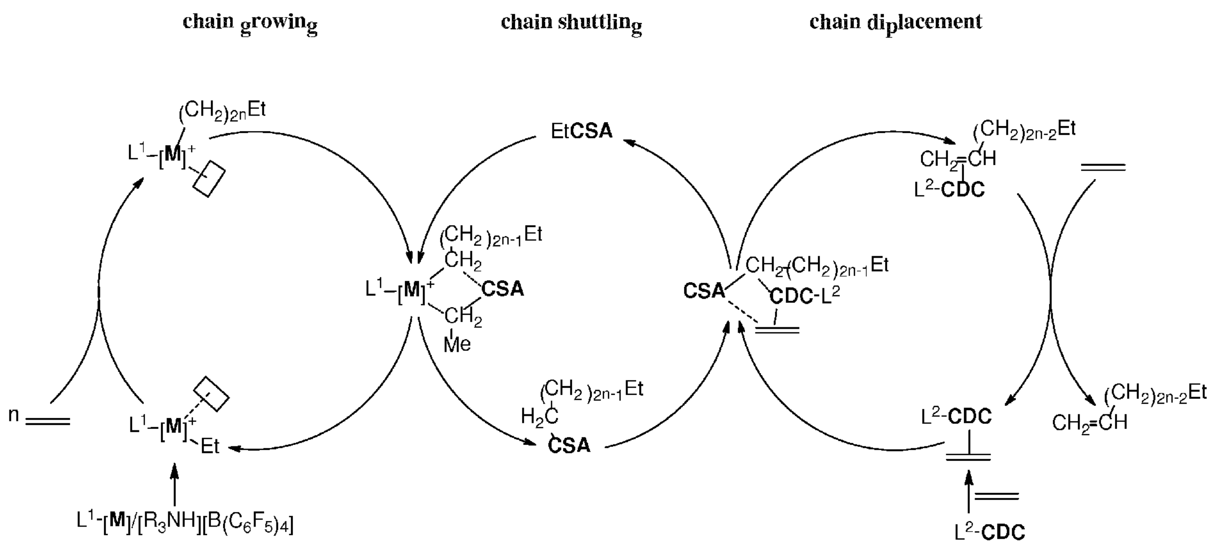

Fig. 2

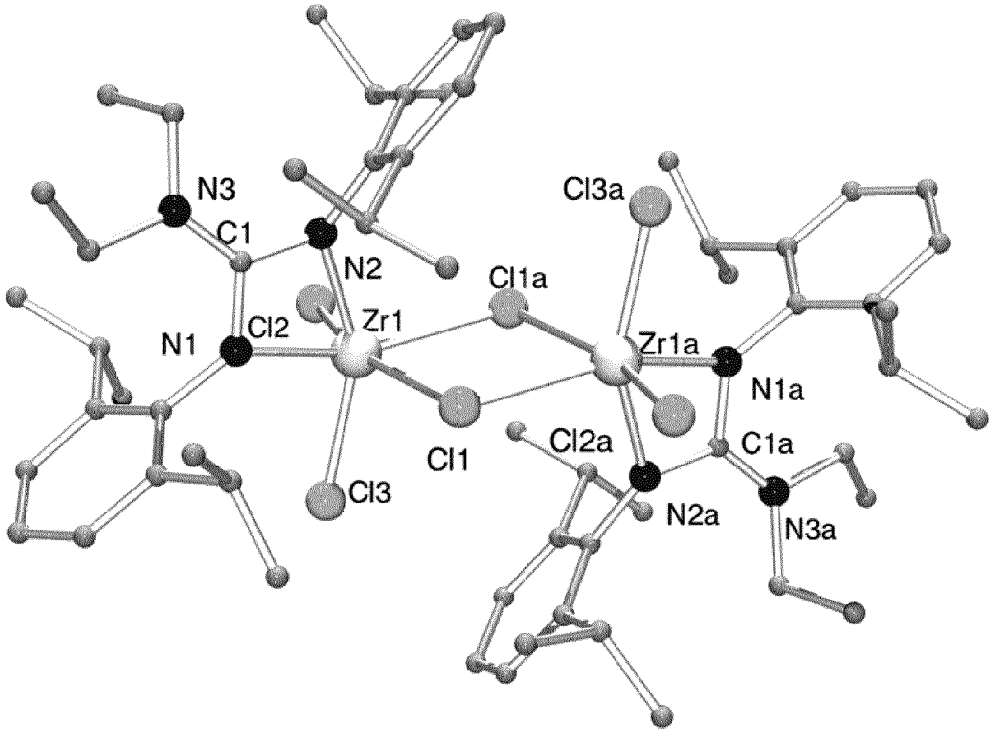

Fig. 3

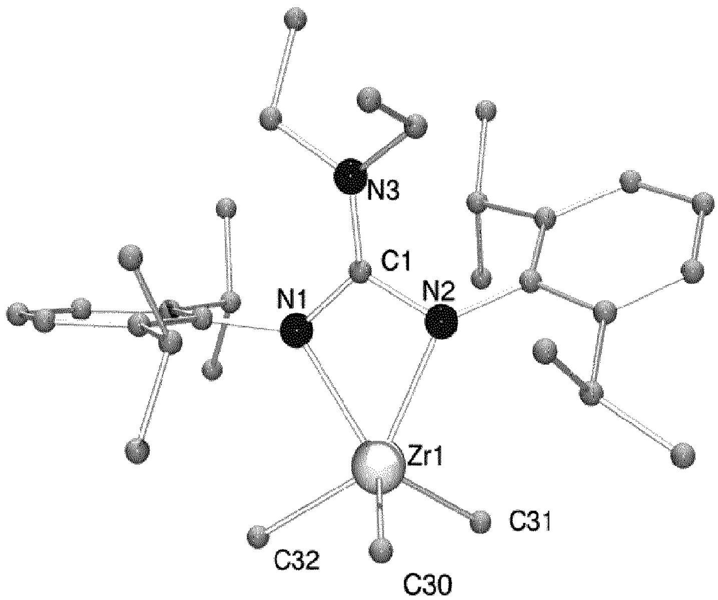

Fig. 4

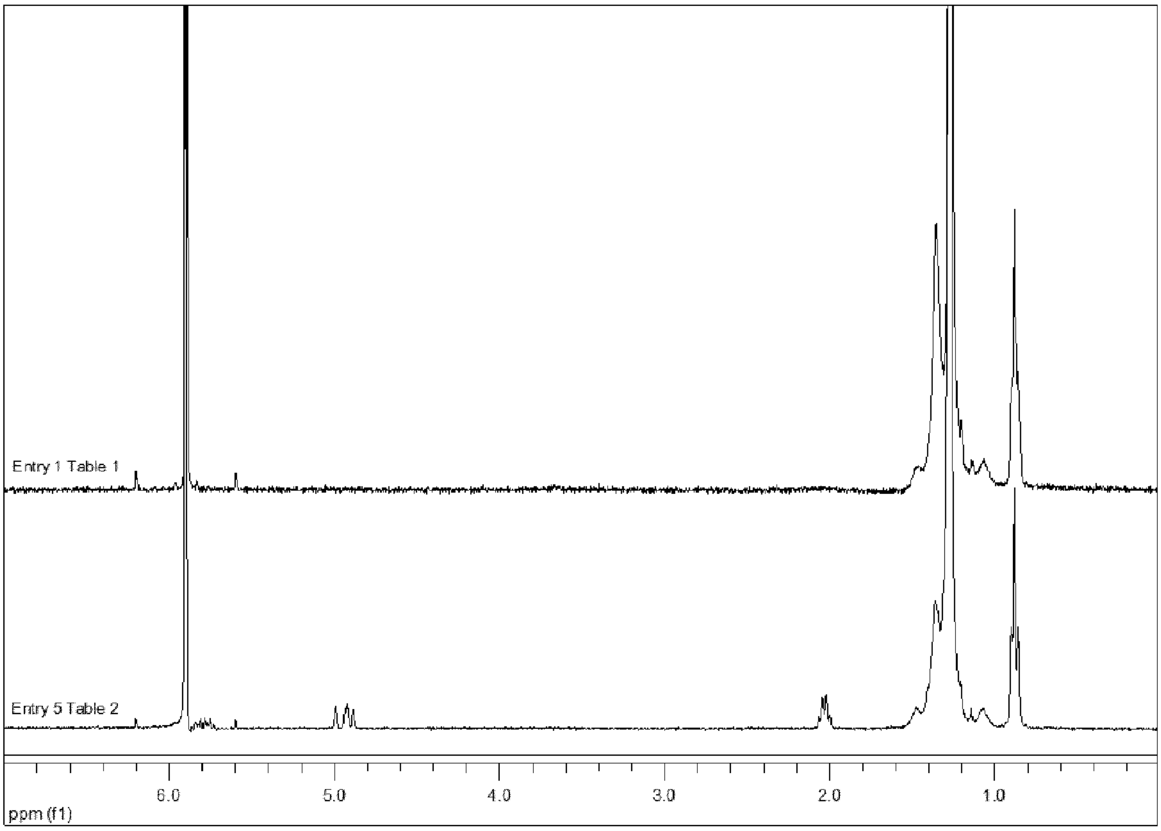

Fig. 5

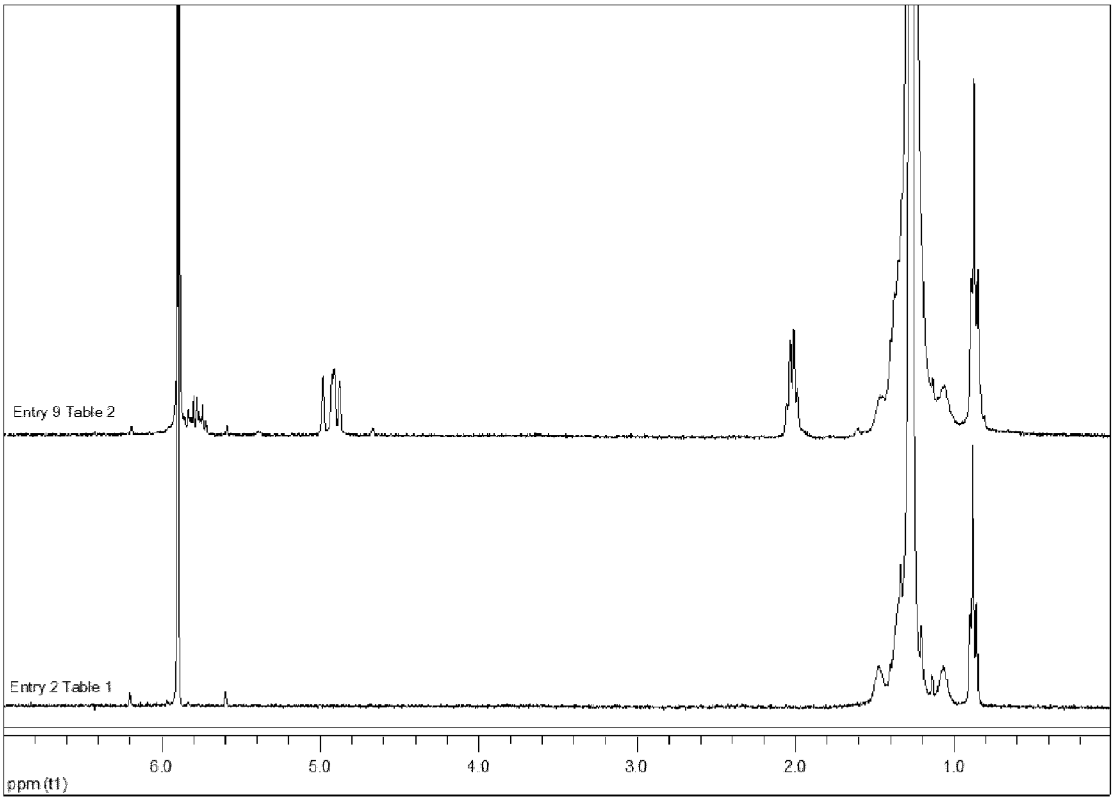

Fig. 6

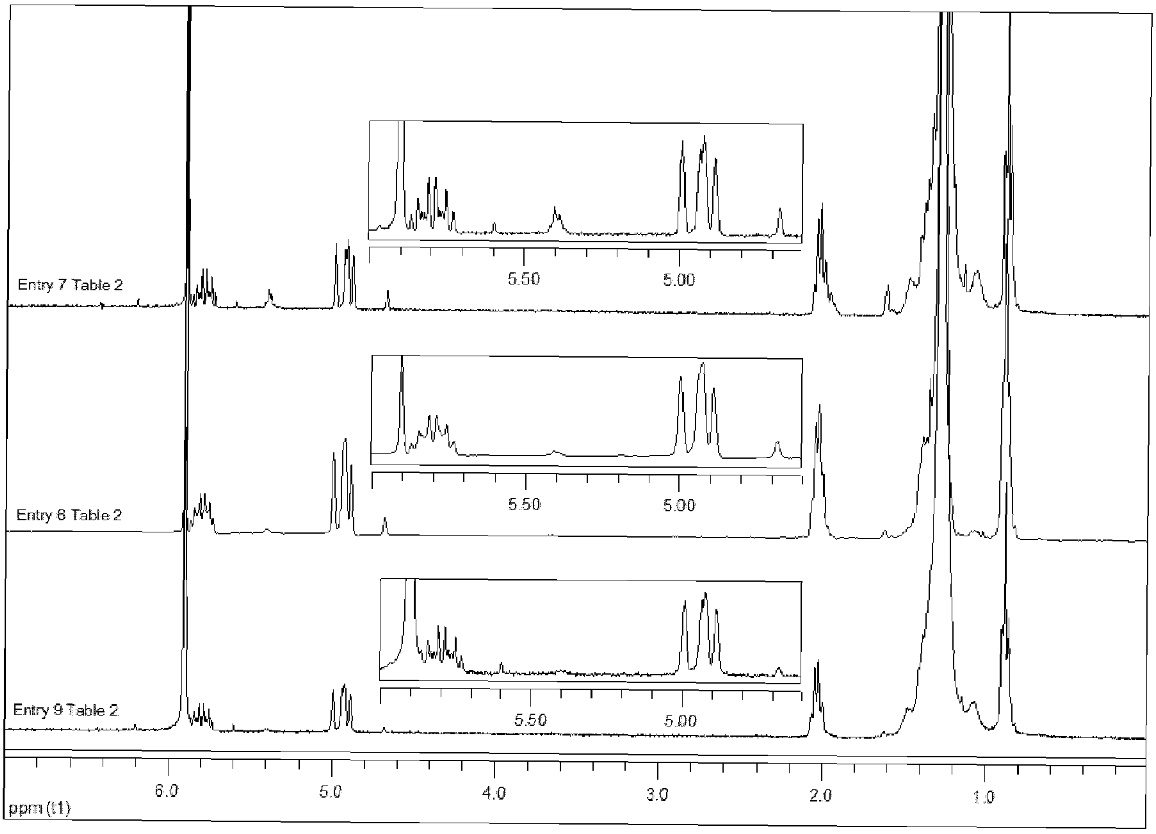
Entry 7 Table 2
5.50
5.00
Entry 6 Table 2
5.50
5.00
Entry 9 Table 2
5.50
5.00
6.0
5.0
4.0
3.0
2.0
1.0
ppm (t1)

Fig. 7

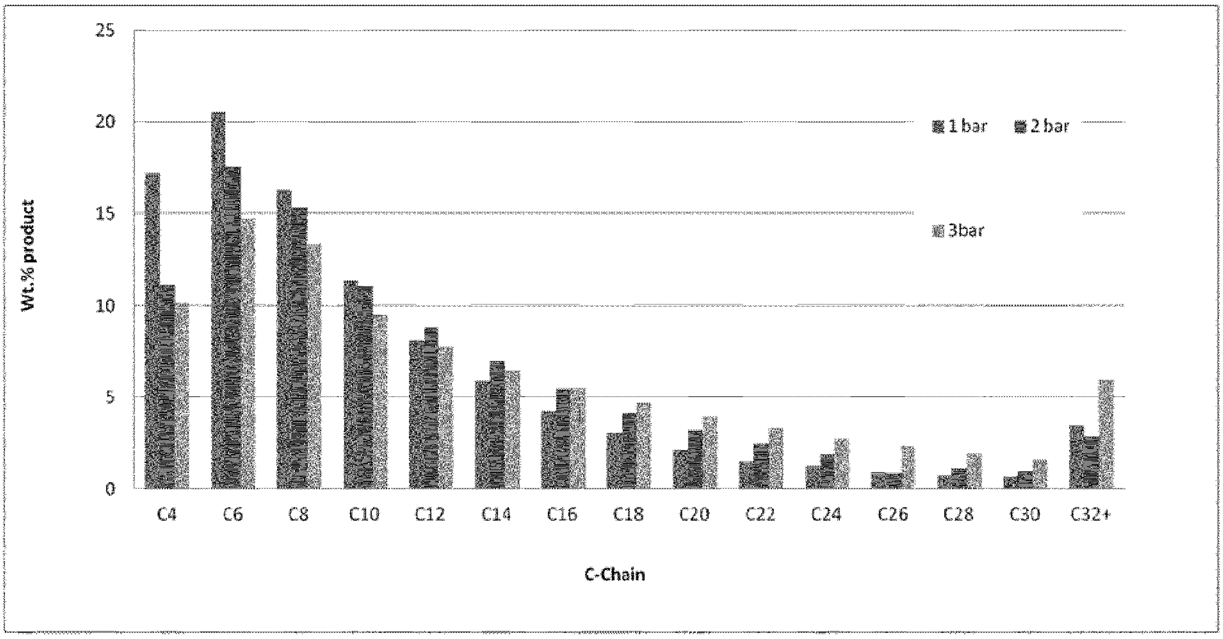
25
20
15
10
5
0
Wt.% product
1 bar
2 bar
3bar
C4
C6
C8
C10
C12
C14
C16
C18
C20
C22
C24
C26
C28
C30
C32+
C-Chain

Fig. 8

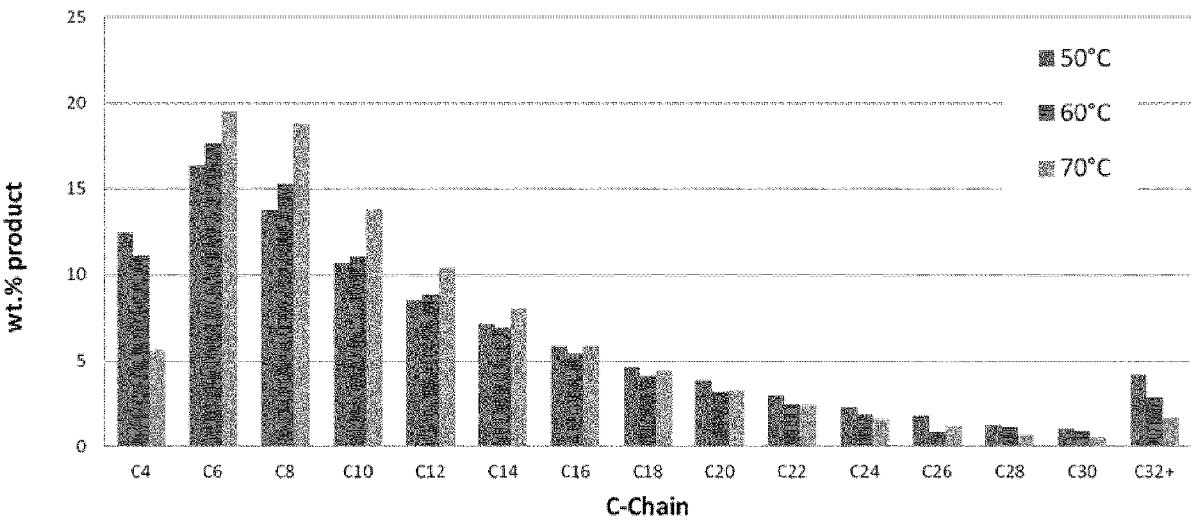
25
20
15
10
5
0
wt.% product
50°C
60°C
70°C
C4
C6
C8
C10
C12
C14
C16
C18
C20
C22
C24
C26
C28
C30
C32+
C-Chain

Fig. 9

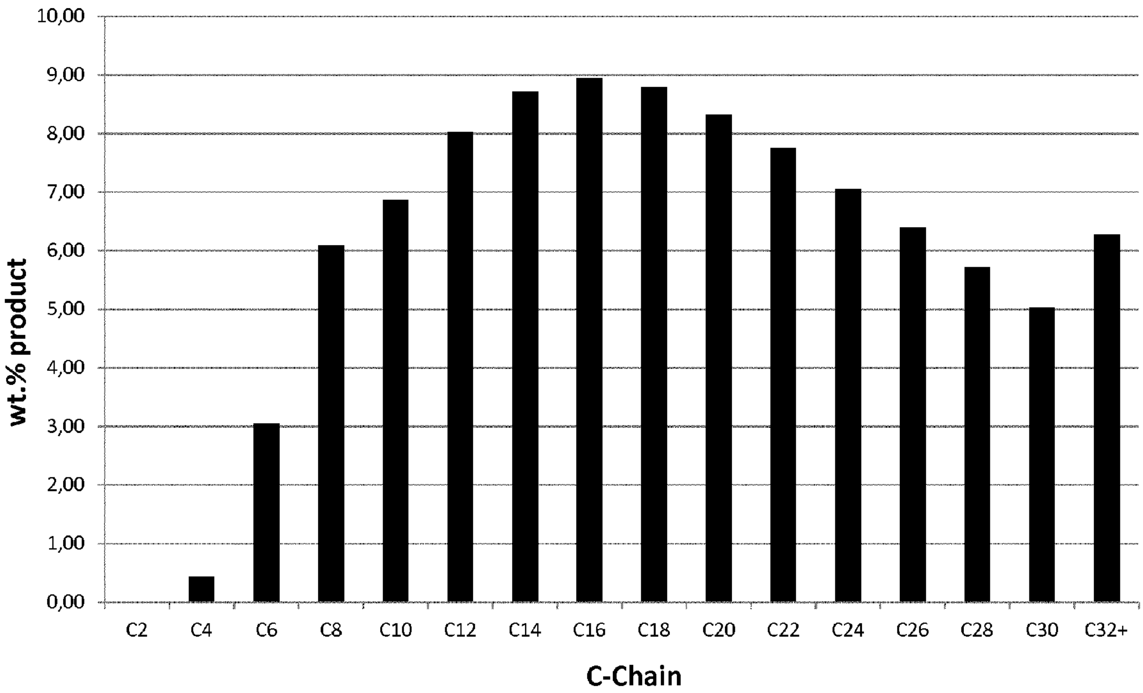
10,00
9,00
8,00
7,00
6,00
5,00
4,00
3,00
2,00
1,00
0,00
wt.% product
C2
C4
C6
C8
C10
C12
C14
C16
C18
C20
C22
C24
C26
C28
C30
C32+
C-Chain

Europäisches Patentamt
European Patent Office
Office européen des brevets

# EUROPEAN SEARCH REPORT

Application Number
EP 15 16 7707

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | EP 2 671 639 A1 (SASOL OLEFINS & SURFACTANTS) 11 December 2013 (2013-12-11)<br>* claims 1, 3, 9, 13; examples *<br>----- | 1-17 | INV.<br>C07C2/32<br>C07C11/02<br>B01J31/12<br>B01J31/14<br>B01J31/18<br>C07F7/00<br>C07C2/88 |
| A,D | US 5 550 303 A (R.L. LIN, ET AL.)<br>27 August 1996 (1996-08-27)<br>* claim 1 *<br>----- | 1,8 | |
| A,D | MEISU ZHOU, ET AL.: "Synthesis, structure and catalytic properties of a novel zirconium guanidinato complex [Zr{ArNC(NMe2)N(SiMe3)}(.mu.2-Cl)Cl2]2[Ar=2,6-i-Pr2-C6H3]",<br>INORGANIC CHEMISTRY COMMUNICATIONS,<br>vol. 10, no. 11,<br>18 October 2007 (2007-10-18), pages 1262-1264, XP022304519,<br>Elsevier, Amsterdam, NL<br>ISSN: 1387-7003, DOI:<br>10.1016/j.inoche.2007.08.001<br>* compound 1 *<br>----- | 1,15-17 | |
| | | | TECHNICAL FIELDS SEARCHED (IPC) |
| | | | C07C<br>B01J |

~~The present search report has been drawn up for all claims~~

1

| Place of search | Date of completion of the search | Examiner |
|---|---|---|
| The Hague | 20 October 2015 | English, Russell |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

Europäisches Patentamt
European Patent Office
Office européen des brevets

Application Number
EP 15 16 7707

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-17

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 15 16 7707

The Search Division considers that the present European patent application does not comply with the requirements of unity of invention and relates to several inventions or groups of inventions, namely:

1. claims: 1-17

   Process of the manufacture of oligomerised olefins.

   ---

2. claims: 18, 19

   Process for the manufacture of di-mu-halogen-bridged bis guanidinato tetra-halogen di zirconium compounds.

   ---

3. claims: 20-22, 24

   Process for the manufacture of zirconium guanidinato alkyl compounds.

   ---

# ANNEX TO THE EUROPEAN SEARCH REPORT ON EUROPEAN PATENT APPLICATION NO. EP 15 16 7707

This annex lists the patent family members relating to the patent documents cited in the above-mentioned European search report. The members are as contained in the European Patent Office EDP file on
The European Patent Office is in no way liable for these particulars which are merely given for the purpose of information.

20-10-2015

| Patent document cited in search report | | Publication date | Patent family member(s) | | | Publication date |
|---|---|---|---|---|---|---|
| EP 2671639 | A1 | 11-12-2013 | CA | 2874682 | A1 | 12-12-2013 |
| | | | CN | 104349840 | A | 11-02-2015 |
| | | | EP | 2671639 | A1 | 11-12-2013 |
| | | | EP | 2855015 | A1 | 08-04-2015 |
| | | | JP | 2015525268 | A | 03-09-2015 |
| | | | US | 2015148505 | A1 | 28-05-2015 |
| | | | WO | 2013182290 | A1 | 12-12-2013 |
| US 5550303 | A | 27-08-1996 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 10022466 A1
- US 20140155666 A1
- US 5276220 A
- WO 2003014046 A1
- EP 2671639 A1
- US 4918254 A
- US 6444867 B
- US 5780697 A
- US 5550303 A
- EP 277003 A
- US 5153157 A
- US 5064802 A
- EP 468651 A
- EP 520732 A


### Non-patent literature cited in the description


- **G. J. P. BRITOVSEK et al.** *Angew. Chem. Int. Ed.,* 1999, vol. 38, 428-447
- **S. D. ITTEL et al.** *Chem. Rev.,* 2000, vol. 100, 1169-1204
- **G. J. P. BRITOVSEK et al.** *Angew. Chem. Int. Ed.,* 2002, vol. 41, 489-491
- **W. P. KRETSCHMER et al.** *Chem. Eur. J.,* 2006, vol. 12, 8969-8978
- **S. B. AMIN ; T. J. MARKS.** *Angew. Chem.,* 2008, vol. 120, 2034-2054
- **I. HAAS et al.** *Organometallics,* 2011, vol. 30, 4854-4861
- **A. VALENTE et al.** *Chem. Rev.,* 2013, vol. 113, 3836-3857
- **S. K. T. PILLAI et al.** *Chem. Eur. J.,* 2012, vol. 18, 13974-13978
- **J. OBENAUF et al.** *Eur. J. Inorg. Chem.,* 2013, 537-544
- **W. P. KRETSCHMER et al.** *Dalton Trans.,* vol. 39, 6847-6852
- **M. BIALEK.** *J. Polym. Sci.: Part A: Polym. Chem.,* 2010, vol. 48, 3209-3214
- **W. P. KRETSCHMER et al.** *Dalton Trans.,* 2010, vol. 39, 6847-6852
- **G. JIN ; C. JONES ; P. C. JUNK ; K.-A. LIPPERT ; R. P. ROSE ; A. STASCH.** *New J. Chem,* 2008, vol. 33, 64-75
- **E. V. AVTOMONOV ; K. A. RUFANOV.** *Z. Naturforsch.,* 1999, vol. 54 b, 1563-1567
- **I, J. OBENAUF ; W. P. KRETSCHMER ; R. KEMPE.** *Eur. J. Inorg. Chem.,* 2014, 1446-1453
- **I, J. OBENAUF ; W. P. KRETSCHMER ; R. KEMPE.** *Eur. J.Inorg. Chem.,* 2014, 1446-1453